# EUROPEAN PATENT APPLICATION

(11) **EP 4 582 412 A1**
(43) Date of publication of application: **09.07.2025**
(21) Application number: 23860911.9
(22) Date of filing: 31.08.2023
(51) Int. Cl.: C07D 207/26, A61K 31/4015, A61P 25/16, B01J 31/02

(54) **CHIRAL GAMMA LACTAM DERIVATIVE OR PHARMACEUTICALLY ACCEPTABLE SALT THEREOF, AND PREPARATION METHOD THEREFOR**

(30) Priority: 31.08.2022 KR 20220110030; 30.08.2023 KR 20230114561
(71) Applicant: Korea University Research and Business Foundation, Seoul 02841 (KR)
(72) Inventor: SHIM, Jae Ho, Seoul 03671 (KR)
(74) Representative: De Clercq & Partners
(86) International application number: PCT/KR2023/012986
(87) International publication number: WO 2024/049236

(57) **Abstract**

The present invention relates to: a method for preparing a chiral gamma lactam derivative or a pharmaceutically acceptable salt thereof by using a chiral organocatalytic compound; and a composition for preventing, alleviating or treating muscle diseases, mental diseases, or neurodegenerative diseases, comprising the derivative or the pharmaceutically acceptable salt thereof. The chiral gamma lactam derivative or the pharmaceutically acceptable salt thereof, of the present invention, has an effect of inhibiting MAO-B and MSTN, targets D1-mClu5, and can be used in the prevention, alleviation, or treatment of muscle diseases including sarcopenia, mental diseases including depression, neurodegenerative diseases including Parkinson's disease, and the like.

## Description

### TECHNICAL FIELD

The present disclosure relates to a chiral gamma-lactam derivative or a pharmaceutically acceptable salt thereof, a method for preparing the same, a composition for preventing, improving, or treating muscle disease, mental disease or degenerative neurological disease including the derivative or a pharmaceutically acceptable salt thereof, etc.

### BACKGROUND ART

Globally, more than 80% of people aged 65 years or over suffer from one or more chronic disease, and more than 68% of them suffer from two or more chronic diseases. A representative example of a chronic disease is Parkinson's disease.

Parkinson's disease is a progressive neurodegenerative disease characterized by symptoms such as slow movement, tremors at rest, muscle rigidity, shuffling gait, and stooped posture. Parkinson's disease shows the morphological characteristic of death of dopamine-producing neurons in the substantia nigra region of the brain. The exact cause of Parkinson's disease has not yet been understood, although it has been linked to the production of reactive oxygen species (ROS), oxidative stress, abnormal formation of protein structures, mitochondrial damage, etc.

As such, when treating Parkinson's disease, the treatment drug must be selected by considering the patient's age, severity of symptoms, and side effects of the drug. It is known that dopamine agonists such as Mirapex or Requip, and MAO-B inhibitors are administered to treat early stage Parkinson's disease, and dopamine agonists such as MAO-B inhibitors, and COMT inhibitors are administered in combination with levodopa drugs to treat late stage Parkinson's disease with motor fluctuations.

Global pharmaceutical companies are developing drugs to treat Parkinson's disease, but most of them have failed in the clinical trial stage. There have been alternative attempts to control the treatment drugs at the receptor or transcription factor level, but the results are not entirely satisfactory. Currently used anti-Parkinson drugs have several fundamental limitations. Most of the anti-Parkinson drugs do not provide a cure, but rather only delay the worsening of symptoms of the disease, primarily by simply quantitatively supplementing the lack of dopamine, which is the main cause of Parkinson's disease symptoms (dopa therapy) or by using dopamine agonists with significant side effects.

Since the underlying pathology of Parkinson's disease is the death of neurons in the substantia nigra of the midbrain, the most fundamental and complete treatment is to suppress the death of dopamine-producing neurons and normalize the dopamine production pathway (neuronal normalization). Meanwhile, existing drugs have consistently shown side effects of dyskinesias and various forms of psychiatric disorders; consequently, they have limitations that prevent them from being a fundamental treatment for the dopaminergic nervous system.

That is, existing treatments for Parkinson's disease have been developed mainly focusing on the mechanism for dopamine agonists and MAO-B inhibitors, and no research has been conducted on the occurrence of muscle atrophy caused by movement disorders in Parkinson's disease. Thus, there are virtually no treatments based on the mechanism for restoring muscle atrophy, both domestically and internationally.

There are approximately 6.3 million Parkinson's disease patients worldwide, and the market is worth approximately 4 trillion won. In Korea, the number of patients exceeds 100,000 and the market is estimated to be worth around 300 billion won. Parkinson's disease has a high incidence rate (approximately 1-2%), especially in the elderly population aged 60 years or over, and as the society is changing into an aging society, it is one of the diseases that has a greater impact on society. Therefore, it is a challenge that must be overcome to realize a healthy society.

Lactams are the main raw materials for medicine and material chemistry with high bioactivity, and are used as very important intermediates in medicine, materials, material chemistry, synthetic chemistry, and the like. Examples include penicillin, the first betalactam antibiotic discovered by humans; zetia, which shows excellent efficacy in treating cardiovascular diseases; levetiracetam, which consists of a gamma-lactam skeleton and is used for treating epilepsy; and azaspirene, which is known for its effect in inhibiting angiogenesis and is used as a treatment for uterine cancer. As such, lactams are physiologically active and widely used as actual medicines, and thus are utilized as the main raw materials for new drug development.

A lactam is a core component of a complex organic molecule and is a basic skeleton widely used in pharmaceuticals, synthetic chemistry, and materials. However, research and development on the catalytic reaction required to produce lactam compounds had been at a standstill for some time. The reason for this was that carbonylnitrene, a key intermediate in the reaction to make lactam compounds from hydrocarbons, broke down into byproducts too easily at room temperature. In other words, the Curtius rearrangement process was the biggest obstacle to lactam production. Therefore, there is an urgent need to develop a new catalyst that may solve this problem.

Under these circumstances, the inventors earnestly and intensively conducted research to solve the problems of the related art described above. As a result, the inventors found that by utilizing a chiral organocatalyst compound with a specific structure, it is possible to produce gamma-lactam derivatives or pharmaceutically acceptable salts thereof of high purity and in large quantities, even using small amounts of catalyst, and that the compounds may be utilized as therapeutic agents that simultaneously enable inhibition of neuronal cell death, neuroprotection, normalization of the dopamine pathway, increased motility, anti-convulsion, and muscle gain. Thereby, the inventors created the present disclosure.

### DISCLOSURE OF THE INVENTION

### TECHNICAL GOALS

A technical goal to be achieved by the present disclosure is to provide a chiral gamma-lactam derivative or a pharmaceutically acceptable salt thereof.

Another technical goal to be achieved by the present disclosure is to provide a composition for preventing, improving or treating muscle disease, mental disease, or degenerative neurological disease, including the derivative or a pharmaceutically acceptable salt thereof as an active ingredient.

Another technical goal of the present disclosure is to provide a method for producing the derivative or a pharmaceutically acceptable salt thereof.

However, the technical goals to be achieved are not limited to those described above, and other goals not mentioned above will be clearly understood by one of ordinary skill in the art from the following description.

### TECHNICAL SOLUTIONS

In order to achieve the above mentioned goals, the present disclosure may provide a pharmaceutical composition for preventing or treating a muscular disease, a mental disease or a degenerative neurological disease, including as an active ingredient any one or more selected from the group consisting of a pharmaceutically acceptable salt of a chiral gamma-lactam derivative represented by the following [Formula 1a]; a pharmaceutically acceptable salt of a chiral gamma-lactam derivative represented by the following [Formula 1b]; a chiral gamma-lactam derivative represented by the following [Formula 1c] or a pharmaceutically acceptable salt thereof; and a combination thereof. In this case, the derivative or a pharmaceutically acceptable salt thereof includes its racemate, solvate, etc.

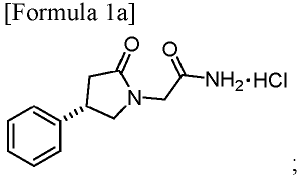

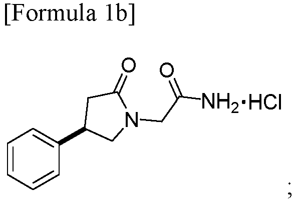

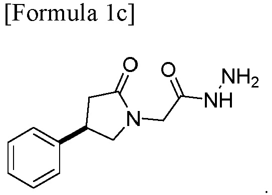

In an embodiment of the present disclosure, the pharmaceutically acceptable salt of the chiral gamma-lactam derivative represented by the following [Formula 1c] may be any one or more selected from the group consisting of hydrochloride salt, bromate, sulfate, phosphate, nitrate, citrate, acetate, lactate, tartrate, maleate, gluconate, succinate, formate, trifluoroacetate, oxalate, fumarate, glutarate, adipate, methanesulfonate, benzenesulfonate, paratoluenesulfonate, camphorsulfonate, sodium salt, potassium salt, lithium salt, calcium salt, magnesium salt, and combinations thereof, but is not limited thereto. Preferably, the pharmaceutically acceptable salt may be a hydrochloride salt, and more preferably, it may be a salt represented by the following [Formula 1d].

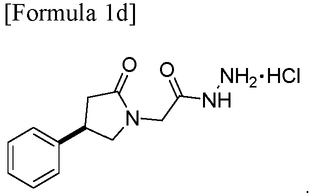

In another embodiment of the present disclosure, the muscular disease may be any one or more selected from the group consisting of atony, muscular atrophy, muscular dystrophy, myasthenia gravis, cachexia, rigid spinesyndrome, amyotrophic lateral sclerosis (Lou Gehrig's disease), Charcot-Marie-Tooth disease, sarcopenia, and combinations thereof, but is not limited thereto.

In another embodiment of the present disclosure, the mental disease may be any one or more selected from the group consisting of depression, mania, bipolar disorder, schizophrenia, delirium, panic disorder, Attention-Deficit Hyperactivity Disorder (ADHD), cognitive disorder, anxiety disorder, Post-Traumatic Stress Disorder (PTSD), and combinations thereof, but is not limited thereto.

In another embodiment of the present disclosure, the degenerative neurological disease may be any one or more selected from the group consisting of Alzheimer's disease, Parkinson's disease, Huntington's disease, Pick's disease, Creutzfeldt-Jakob disease, spinocerebellar degeneration, spinocerebellar ataxia, Friedreich's ataxia, Machado-Joseph disease, dystonia, progressive supranuclear palsy, senile dementia, dementia with Lewy bodies, frontotemporal dementia, vascular dementia, alcohol related dementia, pre-senile dementia, temporal lobe epilepsy, multiple sclerosis, cerebral amyloid angiopathy, systemic amyloidosis, Tourette's disorder, stroke, and combinations thereof, but is not limited thereto.

In addition, the present disclosure may provide the use of any one or more selected from the group consisting of a pharmaceutically acceptable salt of a chiral gamma-lactam derivative represented by the above mentioned [Formula 1a]; a pharmaceutically acceptable salt of a chiral gamma-lactam derivative represented by the above mentioned [Formula 1b]; a chiral gamma-lactam derivative represented by the above mentioned [Formula 1c] or a pharmaceutically acceptable salt thereof; and a combination thereof, for preparing a medicament for preventing or treating muscular disease, mental disease or degenerative neurological disease.

Furthermore, the present disclosure may provide a method for preventing or treating a muscular disease, a mental disease, or a degenerative neurological disease, including a step of administering to a subject any one or more selected from the group consisting of a pharmaceutically acceptable salt of a chiral gamma-lactam derivative represented by the above mentioned [Formula 1a]; a pharmaceutically acceptable salt of a chiral gamma-lactam derivative represented by the above mentioned [Formula 1b]; a chiral gamma-lactam derivative represented by the above mentioned [Formula 1c] or a pharmaceutically acceptable salt thereof; and a combination thereof.

In addition, the present disclosure may provide a cosmetic composition for preventing or improving muscular disease, mental disease or degenerative neurological disease, including as an active ingredient any one or more selected from the group consisting of a cosmetically acceptable salt of a chiral gamma-lactam derivative represented by the above-mentioned [Formula 1a]; a cosmetically acceptable salt of a chiral gamma-lactam derivative represented by the above-mentioned [Formula 1b]; a chiral gamma-lactam derivative represented by the above-mentioned [Formula 1c] or a cosmetically acceptable salt thereof; and a combination thereof.

Furthermore, the present disclosure may provide a food composition for preventing or improving muscular disease, mental disease or degenerative neurological disease, including as an active ingredient any one or more selected from the group consisting of a food-chemically acceptable salt of a chiral gamma-lactam derivative represented by the above-mentioned [Formula 1a]; a food-chemically acceptable salt of a chiral gamma-lactam derivative represented by the above-mentioned [Formula 1b]; a chiral gamma-lactam derivative represented by the above-mentioned [Formula 1c] or a food-chemically acceptable salt thereof; and a combination thereof.

In addition, the present disclosure provides a method for preparing a pharmaceutical composition for preventing or treating a muscular disease, a mental disease, or a degenerative neurological disease, which includes a pharmaceutically acceptable salt of a chiral gamma-lactam derivative represented by [Formula 1a] as an active ingredient, including the following steps:
(1) Producing a compound represented by [Formula 4a] from a compound represented by [Formula 3a] using a catalyst compound represented by [Formula 2];
(2) Producing a compound represented by [Formula 5a] from the compound represented by [Formula 4a];
(3) Producing a compound represented by [Formula 6a] from the compound represented by [Formula 5a]; and
(4) Treating the compound represented by [Formula 6a] with sodium hydride (NaH), ethyl bromoacetate (BrCH₂COOEt), and ammonia (NH₃) to produce a compound represented by [Formula 1a]; 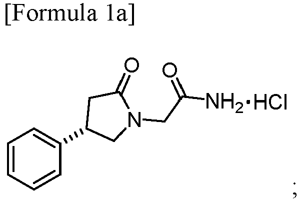 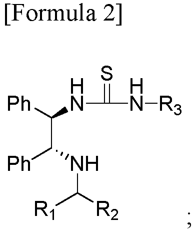 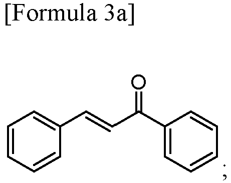 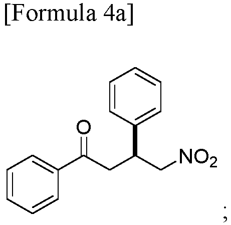 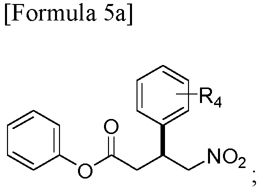 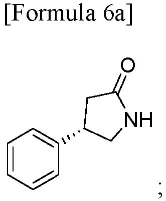
wherein, in Formula 2, R₁ and R₂ are identical or different from each other, and are any one or more selected from the group consisting of hydrogen, a C₁-C₆ alkyl group, a halogen group, a cyano group, a nitro group, a trifluoromethyl group, an alkoxy group, a C₃-C₈ aryl group, and a combination thereof; R₃ is a C₃-C₈ aryl group, wherein the one or more hydrogen of the aryl group is unsubstituted or substituted with any one or more selected from the group consisting of a C₁-C₆ alkyl group, a halogen group, a cyano group, a nitro group, a trifluoromethyl group, an alkoxy group, and a combination thereof; and in Formula 5a, R₄ may be a hydrogen or a halogen group.

In an embodiment of the present disclosure, the catalyst compound represented by the above-mentioned [Formula 2] may be any one or more selected from the group consisting of the catalyst compounds listed in Table 1 below and a combination thereof, but is not limited thereto.

**[Table 1]**

| Formula No. | Compound No. | Compound Name | Formula |
|---|---|---|---|
| 2-1 | 1a | 1-((1R,2R)-2-(3,5-bis(trifluoromethyl)benzylamin o)-1,2-diphenylethyl)-3-(3,5-bis(trifluoromethyl)phenyl)thio urea | 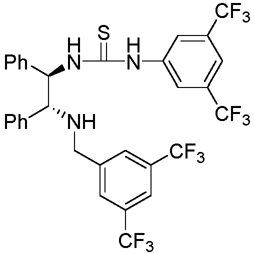 |
| 2-2 | 1b | 1-[(1R,2R)-2-(Pentan-3-ylamino)-1,2-diphenylethyl]-3-(p-tolyl)thiourea | 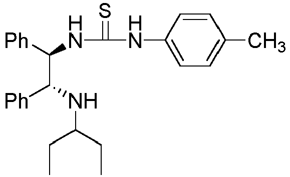 |
| 2-3 | 1c | 1-[3,5-Bis(trifluoromethyl)phenyl]-3-[(1R,2R)-2-(pentan-3-ylamino)-1,2-diphenylethyl]thiourea | 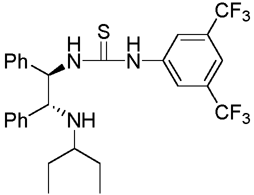 |
| 2-4 | 1d | 1-[(1R,2R)-2-(Pentan-3-ylamino)-1,2-diphenylethyl]-3-[4-(trifluoromethyl) phenyl]thiourea | 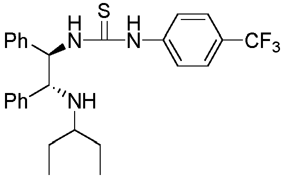 |
| 2-5 | 1e | 1-[(1R,2R)-2-(Pentan-3-ylamino)-1,2-diphenylethyl]-3-(perfluorophenyl)thiourea | 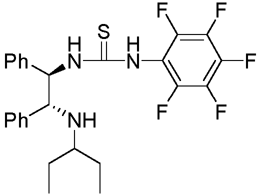 |
| 2-6 | 1f | 1-(4-Nitrophenyl)-3-[(1R,2R)-2-(pentan-3-ylamino)-1,2-diphenylethyl]thiourea | 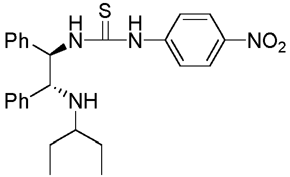 |
| 2-7 | 1g | 1-(4-Cyanophenyl)-3-[(1R,2R)-2-(pentan-3-ylamino)-1,2-diphenylethyl]thiourea | 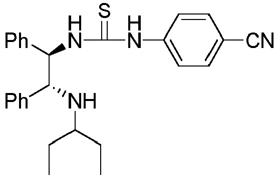 |
| 2-8 | 1h | 1-(4-Fluorophenyl)-3-[(1R,2R)-2-(pentan-3-ylamino)-1,2-diphenylethyl]thiourea | 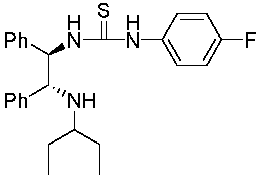 |
| 2-9 | 1i | 1-((1R,2R)-2-(benzhydrylamino)-1,2-diphenylethyl)-3-(3,5-bis(trifluoromethyl)phenyl)thio urea | 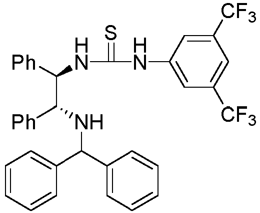 |
| 2-10 | 1j | 1-((1R,2R)-2-(benzhydrylamino)-1,2-diphenylethyl)-3-(4-(trifluoromethyl)phenyl)thioure a | 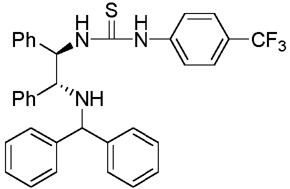 |
| 2-11 | 1k | 1-((1R,2R)-2-(3,5-dimethylbenzylamino)-1,2-diphenylethyl)-3-(3,5-dimethylphenyl)thiourea | 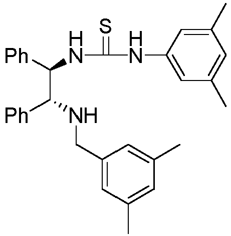 |

In an embodiment of the present disclosure, the compound represented by the above-mentioned [Formula 5a] may be any one or more selected from the group consisting of the compounds listed in Table 2 below and a combination thereof, but is not limited thereto.

**[Table 2]**

| Formula No. | Compound No. | Compound Name | Formula |
|---|---|---|---|
| 5a-1 | 4a | (S)-Phenyl 4-nitro-3-phenylbutanoate | 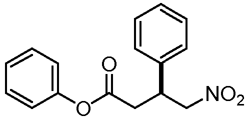 |
| 5a-2 | 4b | (S)-Phenyl 3-(4-chlorophenyl)-4-nitrobutanoate | 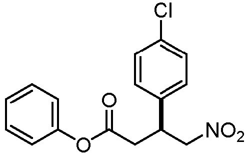 |

In addition, the present disclosure may provide a method for preparing a pharmaceutical composition for preventing or treating a muscular disease, a mental disease, or a degenerative neurological disease, which includes a pharmaceutically acceptable salt of a chiral gamma-lactam derivative represented by [Formula 1b] as an active ingredient, including the following steps:
(1) Producing a compound represented by [Formula 4b] from a compound represented by [Formula 3b] using a catalyst compound represented by [Formula 2];
(2) Producing a compound represented by [Formula 5b] from the compound represented by [Formula 4b];
(3) Producing a compound represented by [Formula 6b] from the compound represented by [Formula 5b]; and
(4) Treating the compound represented by [Formula 6b] with sodium hydride (NaH), ethyl bromoacetate (BrCH₂COOEt), and ammonia (NH₃) to produce a compound represented by [Formula 1b]; 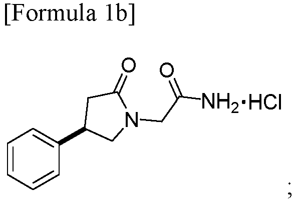 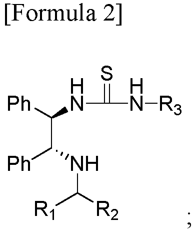 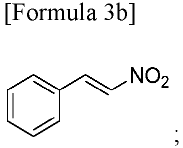 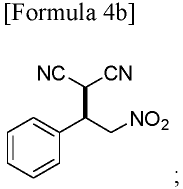 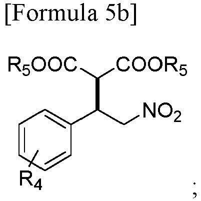 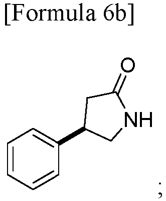
wherein, in Formula 2, R₁ and R₂ are identical or different from each other, and are any one or more selected from the group consisting of hydrogen, a C₁-C₆ alkyl group, a halogen group, a cyano group, a nitro group, a trifluoromethyl group, an alkoxy group, a C₃-C₈ aryl group, and a combination thereof; R₃ is a C₃-C₈ aryl group, wherein the one or more hydrogen of the aryl group is unsubstituted or substituted with any one or more selected from the group consisting of a C₁-C₆ alkyl group, a halogen group, a cyano group, a nitro group, a trifluoromethyl group, an alkoxy group, and a combination thereof; and in Formula 5b, R₄ is a hydrogen or a halogen group, and R₅ may be a C₁-C₆ alkyl group or a benzyl group.

In an embodiment of the present disclosure, the catalyst compound represented by the above-mentioned [Formula 2] may be any one or more selected from the group consisting of the catalyst compounds listed in Table 1 above and a combination thereof, but is not limited thereto.

In an embodiment of the present disclosure, the compound represented by the above-mentioned [Formula 5b] may be any one or more selected from the group consisting of the compounds listed in Table 3 below and a combination thereof, but is not limited thereto.

**[Table 3]**

| Formula No. | Compound No. | Compound Name | Formula |
|---|---|---|---|
| 5b-1 | 2a | (R)-Dimethyl 2-(2-nitro-1-phenylethyl)malonate | 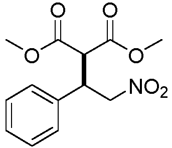 |
| 5b-2 | 2b | (R)-Diethyl 2-(2-nitro-1-phenylethyl)malonate | 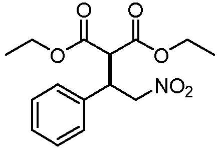 |
| 5b-3 | 2c | (R)-Diisopropyl 2-(2-nitro-1-phenylethyl)malonate | 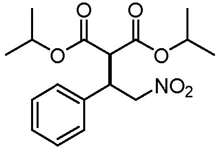 |
| 5b-4 | 2d | (R)-Dipropyl 2-(2-nitro-1-phenylethyl)malonate | 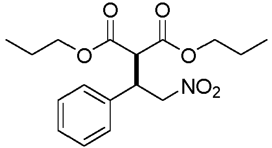 |
| 5b-5 | 2e | (R)-Benzyl-2-carbobenzyloxy-4-nitro-3-phenylbutyrate | 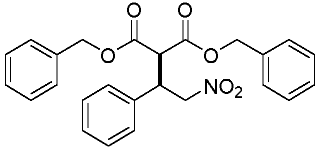 |
| 5b-6 | 2f | (R)-dibutyl 2-(2-nitro-1-phenylethyl)malonate | 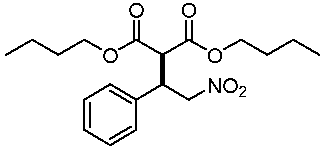 |
| 5b-7 | 2g | (R)-Diethyl 2-[1-(4-bromophenyl)-2-nitroethyl]malonate | 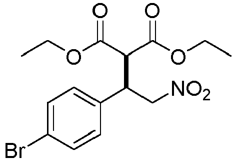 |
| 5b-8 | 2h | (R)-diethyl 2-(1-(4-chlorophenyl)-2-nitroethyl)malonate | 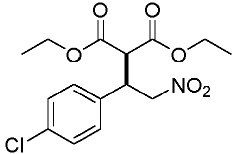 |

In addition, the present disclosure may provide a method for preparing a pharmaceutical composition for preventing or treating a muscular disease, a mental disease, or a degenerative neurological disease, which includes a pharmaceutically acceptable salt of a chiral gamma-lactam derivative represented by [Formula 1d] as an active ingredient, including the following steps:
(1) Producing a compound represented by [Formula 4b] from a compound represented by [Formula 3b] using a catalyst compound represented by [Formula 2];
(2) Producing a compound represented by [Formula 5b] from the compound represented by [Formula 4b];
(3) Producing a compound represented by [Formula 6b] from the compound represented by [Formula 5b]; and
(4) Treating the compound represented by [Formula 6b] with sodium hydride (NaH), ethyl bromoacetate (BrCH₂COOEt), and hydrazine hydrate to produce a compound represented by [Formula 1d]; 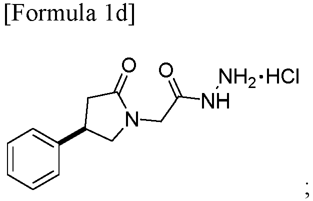 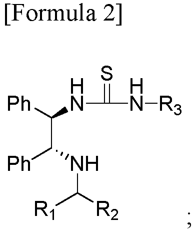 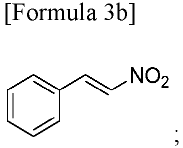 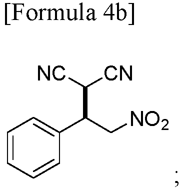 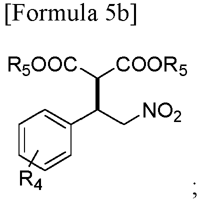 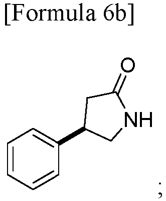
wherein, in Formula 2, R₁ and R₂ are identical or different from each other, and are any one or more selected from the group consisting of hydrogen, a C₁-C₆ alkyl group, a halogen group, a cyano group, a nitro group, a trifluoromethyl group, an alkoxy group, a C₃-C₈ aryl group, and a combination thereof; R₃ is a C₃-C₈ aryl group, wherein the one or more hydrogen of the aryl group is unsubstituted or substituted with any one or more selected from the group consisting of a C₁-C₆ alkyl group, a halogen group, a cyano group, a nitro group, a trifluoromethyl group, an alkoxy group, and a combination thereof; and in Formula 5b, R₄ is a hydrogen or a halogen group, and R₅ may be a C₁-C₆ alkyl group or a benzyl group.

In an embodiment of the present disclosure, the catalyst compound represented by the above-mentioned [Formula 2] may be any one or more selected from the group consisting of the catalyst compounds listed in Table 1 above and a combination thereof, but is not limited thereto.

In an embodiment of the present disclosure, the compound represented by the above-mentioned [Formula 5b] may be any one or more selected from the group consisting of the compounds listed in Table 3 above and a combination thereof, but is not limited thereto.

As used herein, the term "substitution" refers to a reaction in which an atom or atomic group contained in a molecule of a compound is replaced with another atom or atomic group.

As used herein, the term "alkyl group" means a group derived from linear or branched or cyclic saturated aliphatic hydrocarbon having a certain number of carbon atoms and at least one valence. Examples of such alkyl groups include, but are not limited to, methyl, ethyl, propyl, isopropyl, butyl, isobutyl, 2-butyl, 3-butyl, pentyl, n-hexyl, cyclobutyl, cyclopentyl, cyclohexyl groups, etc.

As used herein, the term "aryl group" means an unsaturated aromatic ring compound having 6 to 20 carbon atoms having a single ring (e.g., phenyl) or a plurality of condensed rings (e.g., naphthyl). Examples of such aryl groups include, but are not limited to, phenyl, naphthyl groups, etc.

As used herein, the term "alkoxy group" means an atomic group CnH2n 1O-composed of an oxygen atom bonded to an alkyl group. Examples of such alkoxy groups include, but are not limited to, methoxy, ethoxy, propoxy, or phthoxy groups.

As used herein, the term "halogen group" refers to elements belonging to group 17 of the periodic table, which may include, but are not limited to, fluorine (F), chloride (Cl), bromine (Br), iodine (I), and the like.

### EFFECTS OF THE INVENTION

The chiral gamma lactam derivative or the pharmaceutically acceptable salt thereof, according to an embodiment of the present disclosure, has an effect of inhibiting MAO-B and MSTN, targets D1-mClu5, and may be used in the prevention, alleviation, or treatment of muscle diseases including sarcopenia, mental diseases including depression, neurodegenerative diseases including Parkinson's disease, and the like. In particular, the pharmaceutically acceptable salts may exhibit significantly superior efficacy for controlling Parkinson's disease symptoms compared to the derivatives.

The method for preparing the derivative or a pharmaceutically acceptable salt thereof according to an embodiment of the present disclosure is a chiral small molecule compound-based approach, which is distinct from the conventional approach focusing on dopamine agonists, and thus may preemptively lead the paradigm of new therapeutic agents for treating a muscular disease, a mental disease, or a degenerative neurological disease.

The method for producing the derivative or a pharmaceutically acceptable salt thereof according to one embodiment of the present disclosure enables the production of a chiral gamma-lactam derivative or a pharmaceutically acceptable salt thereof having high stereoselectivity using an appropriate amount of an organic catalyst-based compound. In addition, the chiral gamma-lactam derivatives are key biomaterials and may be discovered as core raw materials for global biopharmaceuticals.

The effects of the chiral gamma lactam derivative or the pharmaceutically acceptable salt thereof according to an embodiment of the present disclosure and a method for producing the same are not limited to the above-mentioned effects. Other unmentioned effects may be clearly understood from the description below by those having ordinary skill in the art to which the present disclosure pertains.

### BRIEF DESCRIPTION OF THE DRAWINGS

FIG. 1 shows the amount of calcium release of Ph-30 (Formula 1b), a pharmaceutically acceptable salt of a chiral gamma-lactam derivative according to one embodiment of the present disclosure, as confirmed by a confocal laser scanning microscope (CLSM).
FIG. 2 shows the calcium release rates of the control rasagiline, and the comparator (R)-phenylpiracetam.
FIG. 3 shows the calcium release rates of the pharmaceutically acceptable salts of chiral gamma-lactam derivatives, Ph-10 (Formula 1d), Ph-20 (Formula 1a), and Ph-30 (Formula 1b) according to one embodiment of the present disclosure.
FIG. 4 shows the effect of pharmaceutically acceptable salts of chiral gamma-lactam derivatives, Ph-10 (Formula 1d), Ph-20 (Formula 1a), and Ph-30 (Formula 1b) according to one embodiment of the present disclosure, on the level of GDF-8/Myostatin expression (*p<0.05. **p<0.01. ***p<0.001).
FIG. 5 shows the effect of pharmaceutically acceptable salts of chiral gamma-lactam derivatives, Ph-10 (Formula 1d), Ph-20 (Formula 1a), and Ph-30 (Formula 1b) according to one embodiment of the present disclosure, on the level of SMAD2 (FIG. 5 (a)) and SMAD3 (FIG. 5 (b)) expression (*p<0.05. **p<0.01).
FIG. 6 shows the results for the treatment of the SH-SY5Y cell line, a human neuroblastoma, with the pharmaceutically acceptable salts of chiral gamma-lactam derivatives, Ph-10 (Formula 1d), Ph-20 (Formula 1a), and Ph-30 (Formula 1b) according to one embodiment of the present disclosure. FIG. 6 (a) shows the expression levels of SYP, GAP-43, MAO-B, and iNOS. FIG. 6 (b) shows the release of calcium, and the production of superoxide anion.
FIG. 7 shows the results of an open field test (OFT) performed by administering the pharmaceutically acceptable salt of a chiral gamma-lactam derivative according to one embodiment of the present disclosure. FIG. 7 (a) shows the distance traveled when Ph-30 (Chemical Formula 1b) was administered according to concentration. FIG. 7 (b) shows the distance traveled when Ph-10 (Formula 1d), Ph-20 (Formula 1a), and Ph-30 (Formula 1b) were administered. (*p<0.05. **p<0.01. ***p<0.001).
FIG. 8 shows the results of a forced swim test (FST) performed by administering the pharmaceutically acceptable salts of chiral gamma-lactam derivatives, Ph-10 (Formula 1d), Ph-20 (Formula 1a), and Ph-30 (Formula 1b) according to one embodiment of the present disclosure (*p<0.05. **p<0.01. ***p<0.001).
FIG. 9 shows the results of a rotarod test performed by administering the pharmaceutically acceptable salts Ph-10 (Chemical Formula 1d), Ph-20 (Chemical Formula 1a), and Ph-30 (Chemical Formula 1b) of a chiral γ-lactam derivative according to one embodiment of the present disclosure. FIG. 9 (a) shows the delay in the time to fall from the accelerating rotarod (ARR). FIG. 9 (b) shows the muscle weight of the tibialis anterior (TA). FIG. 9 (c) shows the muscle weight of the extensor digitorum longus (EDL) (*p<0.05. **p<0.01. ***p<0.001).
FIG. 10 shows the results of analyzing the number of convulsive seizures after administering the pharmaceutically acceptable salts of chiral gamma-lactam derivatives, Ph-10 (Formula 1d), Ph-20 (Formula 1a), and Ph-30 (Formula 1b) according to one embodiment of the present disclosure (**p<0.01).
FIG. 11 shows the effect of pharmaceutically acceptable salts of chiral gamma-lactam derivatives, Ph-10 (Formula 1d), Ph-20 (Formula 1a), and Ph-30 (Formula 1b) according to one embodiment of the present disclosure, on the level of GDF-8/Myostatin expression in mouse serum (*p<0.05. **p<0.01).

### BEST MODE FOR CARRYING OUT THE INVENTION

In the conventional synthesis of gamma-lactam derivatives using chiral catalysts, the inventors have solved the problems of the difficulty in chiral catalyst synthesis, excessive use of chiral catalysts, etc.; and they have solved the problem of mass production through shortening the reaction time.

Specifically, the inventors prepared an unnatural gamma-lactam derivative or a pharmaceutically acceptable salt thereof with high stereoselectivity from nitro compounds using a bifunctional chiral organocatalyst compound with excellent stereoselectivity. Furthermore, the above mentioned synthesized chiral gamma-lactam derivatives or pharmaceutically acceptable salts thereof were tested for MAO-B and MSTN inhibitory activity, and for effectiveness in preventing, ameliorating, or treating muscular diseases, metal diseases, or degenerative neurological diseases through behavioral experiments and brain activity alteration studies.

Based on the results mentioned above, the present disclosure provides a pharmaceutical composition for preventing or treating muscular diseases, mental diseases, or degenerative neurological diseases, including the chiral gamma-lactam derivatives or pharmaceutically acceptable salts thereof listed in Table 4 below as active ingredients.

**[Table 4]**

| Formula No. | Compound No. | Compound Name | Formula |
|---|---|---|---|
| 1a | 5a (Ph-20) | (S)-2-(2-oxo-4-phenylpyrrolidin-1-yl)acetamide hydrochloride | 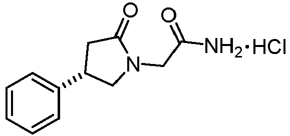 |
| 1b | 5b (Ph-30) | (R)-2-(2-oxo-4-phenylpyrrolidin-1-yl)acetamide hydrochloride | 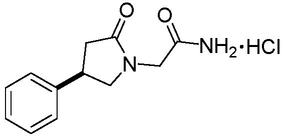 |
| 1c | | (R)-2-(2-oxo-4-phenylpyrrolidin-1-yl)acetohydrazide | 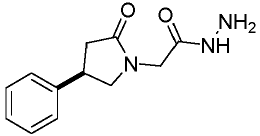 |
| 1d | 5c (Ph-10) | (R)-2-(2-oxo-4-phenylpyrrolidin-1-yl)acetohydrazide hydrochloride | 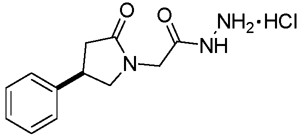 |

In addition, the present disclosure may provide the use of chiral gamma-lactam derivatives or pharmaceutically acceptable salts thereof for the preparation of a medicament for preventing or treating a muscular disease, a mental disease, or a degenerative neurological disease.

Furthermore, the present disclosure may provide a method for preventing or treating a muscular disease, a mental disease, or a degenerative neurological disease, including a step of administering the chiral gamma-lactam derivatives or pharmaceutically acceptable salts thereof to a subject.

As used herein, the term "pharmaceutically acceptable salt" means a formulation of a compound that does not cause serious irritation to the organism to which the compound is administered and does not impair the biological activity and properties of the compound. The pharmaceutically acceptable salt of the compound of the present disclosure may be obtained by reacting the compound of the present disclosure with inorganic acids such as hydrochloric acid, hydrobromic acid, sulfuric acid, nitric acid, and phosphoric acid; sulfonic acids such as methanesulfonic acid, ethanesulfonic acid, and p-toluenesulfonic acid; organic carboxylic acids such as tartaric acid, formic acid, citric acid, acetic acid, trichloroacetic acid, trifluoroacetic acid, capric acid, isobutanoic acid, malonic acid, succinic acid, phthalic acid, gluconic acid, benzoic acid, lactic acid, fumaric acid, maleic acid, and salicylic acid. In addition, the pharmaceutically acceptable salt of the compound of the present disclosure may be obtained by reacting the compound of the present disclosure with a base to form salts such as ammonium salt, alkali metal salt such as sodium or potassium salt, alkaline earth metal salt such as calcium or magnesium salt, salts of organic bases such as dicyclohexylamine, N-methyl-D-glucamine, and tris(hydroxymethyl)methylamine, and salts of amino acids such as arginine and lysine. Furthermore, the derivative or a pharmaceutically acceptable salt thereof of the present disclosure may include not only pharmaceutically acceptable salts, but also all salts, hydrates and solvates that may be prepared by conventional methods.

As used herein, the term "prevention" refers to all actions that inhibit or delay the occurrence, spread, or recurrence of a geriatric disease, such as muscular disease, depression, or Parkinson's disease, by the administration of the composition of the present disclosure, and the term "treatment" refers to all actions by which the symptoms of the disease are ameliorated or beneficially changed by the administration of a composition of the present disclosure.

As used herein, the term "pharmaceutical composition" means a composition manufactured for the prevention or treatment of the disease, which may be formulated and used in various forms according to conventional methods. For example, the composition may be formulated in oral dosage forms such as powders, granules, tablets, capsules, suspensions, emulsions, syrups, and the like, and may be formulated and used in the form of external preparations, suppositories, and sterile injectable solutions.

As used herein, the phrase "including as an active ingredient" means that the ingredient is included in an amount necessary or sufficient to realize a desired biological effect. In practical application, the amount to be included as an active ingredient may be determined to be an amount to treat the disease of interest and not cause other toxicities. For example, the amount may vary depending on a variety of factors, such as the disease or condition being treated, the form of the composition being administered, the size of the individual, or the severity of the disease or condition. A person of ordinary skill in the art to which the present disclosure pertains will be able to determine empirically the effective amount of an individual composition without undue experimentation.

In addition, the pharmaceutical composition of the present disclosure may, depending on each formulation, further include one or more pharmaceutically acceptable carriers, in addition to the active ingredients described above.

The pharmaceutically acceptable carrier may be saline, sterile water, Ringer's solution, buffered saline, dextrose solution, maltodextrin solution, glycerol, ethanol, and a mixture of one or more of these components, and may further include other conventional additives, such as antioxidants, buffers, bacteriostatic agents, and the like. In addition, the composition may be formulated in injectable forms, such as aqueous solutions, suspensions, emulsions, etc., pills, capsules, granules or tablets by additionally adding diluents, dispersants, surfactants, binders and lubricants. Further, the composition may be preferably formulated depending on the disease or ingredient, by any suitable method in the art, or as disclosed in Remington's Pharmaceutical Science (Mack Publishing Company, Easton PA).

The pharmaceutical composition of the present disclosure may be administered orally or parenterally in a pharmaceutically effective amount, depending on the intended method, and the term "pharmaceutically effective amount" as used herein means an amount sufficient to treat a disease at a reasonable benefit/risk ratio applicable to medical treatment and not causing side effects. The effective dose level may be determined based on factors including the patient's health condition, severity, activity of the drug, sensitivity to the drug, method of administration, time of administration, route of administration and rate of elimination, duration of treatment, drugs used in combination or concurrently, and other factors well known in the medical field.

As used herein, the term "individual" refers to, but is not limited to, any mammal, such as a domestic animal or human in need of prevention, treatment, and/or diagnosis of the diseases, but preferably a human.

The pharmaceutical composition of the present disclosure may be formulated in various forms for administration to an individual, and a representative form for parenteral administration is an injectable form, preferably an isotonic aqueous solution or suspension. The injectable form may be prepared by techniques known in the art using a suitable dispersing or wetting agent and a suspending agent. For example, each component may be dissolved in a saline or buffer solution, and then the mixture may be formulated for injection. The formulations for oral administration include, for example, swallowable tablets, buccal tablets, troches, capsules, elixirs, suspensions, syrups, and wafers, which may include, in addition to an active ingredient, a diluent (e.g., lactose, dextrose, sucrose, mannitol, sorbitol, cellulose, and/or glycine) and a lubricant (e.g., silica, talc, stearic acid and its magnesium or calcium salt, and/or polyethylene glycol). The tablets may include a binder such as magnesium aluminum silicate, starch paste, gelatin, tragacanth, methylcellulose, sodium carboxymethylcellulose, and/or polyvinylpyrrolidine, and in some instances, may further include disintegrating agents such as starch, agar, alginic acid or its sodium salts, absorbents, colorants, flavoring agents, and/or sweeteners. The formulations may be prepared by conventional mixing, granulating, or coating methods.

Further, the pharmaceutical composition of the present disclosure may additionally contain adjuvants such as preservatives, hydrating agents, emulsification accelerators, salts or buffers for osmotic pressure control, and other therapeutically useful substances, and may be formulated according to a conventional method.

The pharmaceutical composition of the present disclosure may be administered via several routes, including oral, transdermal, subcutaneous, intravenous, and intramuscular, and the dosage of the active ingredient may be adequately selected depending on several factors, including the route of administration, the age, gender, and weight of the patient, and the severity of the patient's disease. Additionally, the pharmaceutical composition of the present disclosure may be administered in combination with known compounds, which may enhance the desired effect.

The pharmaceutical composition of the present disclosure may be administered to humans and animals orally or parenterally, for example, intravenously, subcutaneously, intranasally or intraperitoneally. Oral administration also includes sublingual application. Parenteral administration includes injections and drop methods such as subcutaneous injection, intramuscular injection, and intravenous injection.

In addition, the pharmaceutical composition according to the present disclosure is not particularly limited in terms of formulation, route of administration and method of administration, so long as it exhibits the effects of the present disclosure, and may additionally include known medicaments in addition to the chiral gamma-lactam derivative or a pharmaceutically acceptable salt thereof as an active ingredient, and may also be used in combination with other known therapies for the treatment of these diseases.

The terminology used herein is for the purpose of describing particular embodiments only and is not intended to be limiting. The singular forms "a, " "an," and "the" are intended to include the plural forms as well, unless the context clearly indicates otherwise. It will be further understood that the terms "comprises/comprising" and/or "includes/including" when used herein, specify the presence of stated features, integers, steps, operations, elements, and/or components, but do not preclude the presence or addition of one or more other features, integers, steps, operations, elements, components and/or groups thereof.

Unless otherwise defined, all terms including technical and scientific terms used herein have the meaning commonly understood by one of ordinary skill in the art to which the examples belong. It will be further understood that terms defined in commonly used dictionaries, should be interpreted as having a meaning that is consistent with the meaning in the context of the relevant art and should not be interpreted in an idealized or overly formal sense unless expressly so defined herein.

In addition, terms such as first, second, A, B, (a), (b), and the like may be used to describe components of the embodiments. These terms are used only for the purpose of discriminating one constituent element from another constituent element, and do not limit the nature, the sequences, or the orders of the constituent elements. It should be noted that if one component is described as being "connected," "coupled" or "joined" to another component, the former may be directly "connected," "coupled," and "joined" to the latter or "connected", "coupled", and "joined" to the latter via another component.

### MODE FOR CARRYING OUT THE INVENTION

Hereinafter, embodiments will be described in detail with reference to the accompanying drawings. However, since various changes may be made to the embodiments, it will be understood that the descriptions of the embodiments do not limit or otherwise restrict the scope of the patent application. With respect to the embodiments, it will be understood that the scope of the present disclosure shall include all changes, equivalents, and replacements.

In addition, in the descriptions of the embodiments making reference to the accompanying drawings, like reference numerals refer to like constituent elements and any repeated description related thereto has been omitted. In the descriptions of the embodiments, any detailed description of well-known related art has been omitted where it is deemed that such description will cause ambiguous interpretation of the present disclosure.

Now, specific embodiments will be described in detail with reference to the accompanying drawings, although the present disclosure may have various embodiments and various changes may be made to the embodiments. However, such illustrations and descriptions are not intended to limit the present disclosure to specific embodiments, and should be understood as including all transformations, equivalents, and substitutes that may be included in the spirit and scope of the present disclosure. In the description of the present disclosure, any detailed description of well-known related structures or functions has been omitted where it is deemed that such description will cause ambiguous interpretation of the present disclosure.

### Example 1. Preparation of chiral organocatalyst compound of the present disclosure

A method for the preparation of an organochiral catalyst compound, namely an arylated thiourea catalyst, according to one embodiment of the present disclosure is shown by Scheme 1 below.

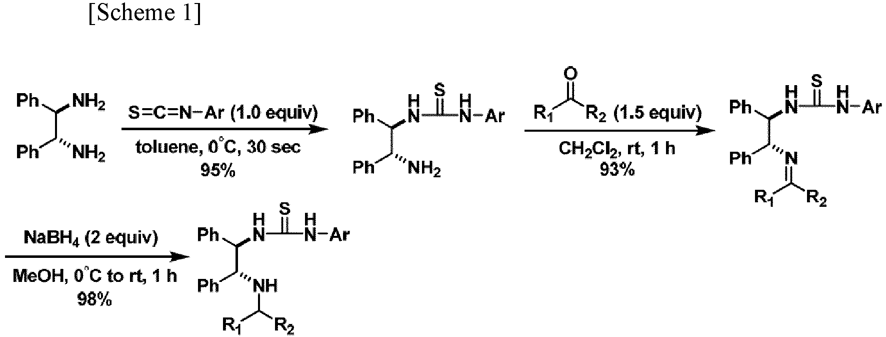

Thiourea (0.95 eq) was added to a suspension of (R,R)-1,2-diphenylethylenediamine (1.0 eq) in toluene (0.5 M) at 0°C, and the mixture was stirred for 30s. The product was concentrated in vacuo and purified by flash column chromatography on silica gel containing ethyl(methanol/methylene chloride 1:20). Alkyl ketone (1.1 equiv) was added to thiourea-substituted DPEN (1.0 eq) in CH₂Cl₂ (0.1 M), and the mixture was stirred at room temperature for 1hour. NaBH₄ (2.0 eq) and ethanol were added thereto at 0°C, and the mixture was stirred at room temperature for 1 hour. The product was filtered through a Celite filter, and the mixture was extracted with ethyl acetate. The combined organic extracts were washed with brine, dried over MgSO₄, and vacuum concentrated. The product was purified by chromatography on a silica gel column (methanol/methylene chloride 1:20) to obtain the pure amide product (quantitative yield) as a brown foamy solid.

Specific examples of the above mentioned chiral organocatalyst compounds are shown in Table 5 below.

**[Table 5]**

| Formula No. | Compound No. | R₁ | R₂ | R₃ |
|---|---|---|---|---|
| 2-1 | 1a | H | 3,5-(CF₃)₂-Ph | 3,5-(CF₃)₂-Ph |
| 2-2 | 1b | ethyl | ethyl | 4-CH₃-Ph |
| 2-3 | 1c | ethyl | ethyl | 3,5-(CF₃)₂-Ph |
| 2-4 | 1d | ethyl | ethyl | 4-CF₃-Ph |
| 2-5 | 1e | ethyl | ethyl | C₆F₅ |
| 2-6 | 1f | ethyl | ethyl | 4-NO₂-Ph |
| 2-7 | 1g | ethyl | ethyl | 4-CN-Ph |
| 2-8 | 1h | ethyl | ethyl | 4-F-Ph |
| 2-9 | 1i | phenyl | phenyl | 3,5-(CF₃)₂-Ph |
| 2-10 | 1j | phenyl | phenyl | 4-CF₃-Ph |
| 2-11 | 1k | H | 3,5-(CH₃)₂-Ph | 3,5-(CH₃)₂-Ph |

(1) 1-((1R,2R)-2-(3,5-bis(trifluoromethyl)benzylamino)-1,2-diphenylethyl)-3-(3,5-bis(trifluoromethyl)phenyl)thiourea (**1a**, Formula 2-1)
   93% yield; [α]_{D}²⁰=+0.45 (c=0.11, CH₂Cl₂); ¹H NMR (500 MHz, DMSO-d₆) δ7.61 (br, 3 H), 7.39~7.29 (m, 16 H), 4.54 (s, 4 H); ¹³C NMR (100 MHz, DMSO-d₆) δ 171.58, 157.99, 142.12,131.46, 134.14, 129.17, 127.28, 125.67, 122.96, 112.29, 89.59, 89.05, 84.78; IR (KBr) 3032.6, 2871.3, 1663.5, 1386.6, 1275.9, 1117.5, 930.2, 700.2 cm⁻¹; HRMS (FAB⁺) for C₃₂H₂₃F₁₂N₃S [M+H]⁺ Calcd: 709.1421, Found: 709.1428
(2) 1-[(1R,2R)-2-(Pentan-3-ylamino)-1,2-diphenylethyl]-3-(p-tolyl)thiourea (**1b**, Formula 2-2)
   86% yield; [α]_{D}²⁰=+0.19 (c=1.00, CH₂Cl₂); ¹H NMR (300 MHz, DMSO-d₆) δ9.76 (s, 1 H), 7.89 (d, J=7.0 Hz, 1 H), 7.32~7.18 (m, 14 H), 5.44 (s, 1 H), 4.08 (d, J=5.1 Hz, 1 H), 2.29 (s, 2 H), 2.02 (s, 1 H), 1.39 (s, 1 H), 1.20~1.06 (m, 4 H), 0.68 (t, J=7.5 Hz, 3 H), 0.41 (t, J=7.1 Hz, 3 H); ¹³C NMR (100 MHz, DMSO-d₆) δ181.04, 141.83, 141.55, 136.71, 134.88, 130.03, 128.67, 128.51, 127.49, 127.40, 124.67, 64.28, 63.77, 55.84, 26.71, 24.02, 21.20, 10.94, 8.30; IR (KBr) 3180.2, 2958.4, 1948.8, 1510.1, 1240.1, 821.6, 700.1, 565.1 cm⁻¹; HRMS (FAB⁺) for C₂₇H₃₄N₃S [M+H]⁺ Calcd: 432.2473, Found: 432.6537, pattern 432.5, 345.3, 266.4, 176.3, 106.01
(3) 1-[3,5-Bis(trifluoromethyl)phenyl]-3-[(1R,2R)-2-(pentan-3-ylamino)-1,2-diphenylethyl]thiourea (**1c**, Formula 2-3)
   90% yield; [α]_{D}²⁰=+0.31 (c=0.11, CH₂Cl₂); ¹H NMR (300 MHz, DMSO-d₆) δ10.5 (br, 1 H), 8.30 (s, 2 H), 7.74 (s, 1 H), 7.40~7.19 (m, 10 H), 5.57 (br, 1 H), 4.18 (d, J=4.9 Hz, 1 H), 2.09 (m, 1 H), 1.24~1.20 (m, 4 H), 0.75 (t, J=7.1 Hz, 3 H), 0.50 (t, J=6.0 Hz, 3 H); ¹³C NMR (100 MHz, DMSO-d₆) δ181.10, 142.49, 140.88, 130.96, 130.64, 128.70, 128.59, 128.56, 127.60, 125.22, 122.52, 122.19, 116.70, 64.34, 63.62, 56.48, 26.64, 23.90, 10.98, 8.54; IR (KBr) 3239.9, 2964.2, 1471.5, 1278.6, 1135.9, 885.2, 700.1 cm⁻¹; HRMS (FAB⁺) for C₂₈H₃₀F₆N₃S [M+H]⁺ Calcd: 554.2065, Found: 554.2065
(4) 1-[(1R,2R)-2-(Pentan-3-ylamino)-1,2-diphenylethyl]-3-[4-(trifluoromethyl) phenyl]thiourea (**1d**, Formula 2-4)
   88% yield; [α]_{D}²⁰=+45.5 (c=0.02, CH₂Cl₂); ¹H NMR (300 MHz, DMSO-d₆) δ10.2 (br s, 1H), 8.41 (br s, 1H), 7.79 (d, J=8.0 Hz, 2H), 7.64 (d, J=8.5 Hz, 2H), 7.35~7.15 (m, 10H), 5.53 (br s, 1H), 4.13 (d, J=5.5 Hz, 1H), 2.07 (m, 1H), 1.30~1.15 (m, 4H), 0.73 (t, J=7.1 Hz, 3H), 0.49 (t, J=6.9 Hz, 3H); ¹³C NMR (100 MHz, DMSO-d₆) δ180.91, 143.97, 141.18, 128.64, 128.52, 127.69, 127.48, 126.29, 122.41, 64.43, 63.71, 56.32, 26.68, 23.98, 10.98, 8.53; IR(KBr) 3205.3, 2962.3, 1945.9, 1741.5, 1517.8, 1324.9, 1245.9, 1066.5, 840.9, 700.1, 597.9 cm⁻¹; HRMS(FAB⁺) for C₂₇H₃₁F₃N₃S[M+H]⁺ Calcd: 486.2191, Found: 486.2190
(5)1-[(1R,2R)-2-(Pentan-3-ylamino)-1,2-diphenylethyl]-3-(perfluorophenyl)thiourea (**1e**, Formula 2-5)
   89% yield; [α]_{D}²⁰=+80.4 (c=0.02, CH₂Cl₂); ¹H NMR (300 MHz, DMSO-d₆) δ9.47 (s, 1 H), 8.61 (s, 1 H), 7.30~7.15 (m, 10 H), 5.48 (br s, 1 H), 4.13 (d, J=6.1 Hz, 1 H), 2.08 (m, 1 H), 1.54 (br, 1 H), 1.30~1.14 (m, 4 H), 0.74 (t, J=7.4 Hz, 3 H), 0.55 (t, J=6.3 Hz, 3 H); ¹³C NMR (100 MHz, DMSO-d₆) δ183.63, 145.82, 143.43, 141.90, 140.77, 139.01, 138.84, 136.56, 129.39, 128.61, 128.43, 127.68, 127.60, 115.93, 64.77, 64.53, 56.37, 26.72, 24.16, 10.86, 8.58; IR (KBr) 3299.8, 2964.2, 1525.5, 1344.2, 1145.6, 991.3, 912.2, 700.1, 605.6 cm⁻¹; HRMS (FAB⁺) for C₂₆H₂₇F₅N₃S [M+H]⁺ Calcd: 508.1846, Found: 508.1848
(6) 1-(4-Nitrophenyl)-3-[(1R,2R)-2-(pentan-3-ylamino)-1,2-diphenylethyl]thiourea (**1f**, Formula 2-6)
   89% yield; [α]_{D}²⁰=+37.7 (c=0.02, CH₂Cl₂); ¹H NMR (300 MHz, DMSO-d₆) δ10.5 (s, 1 H), 8.16 (m, 2 H), 7.90 (d, J=9.1 Hz, 2 H), 7.37~7.15 (m, 10 H), 5.54 (br s, 1 H), 4.16 (d, J=5.5 Hz, 1 H), 2.07 (m, 1 H), 1.30~1.15 (m, 4 H), 0.75 (t, J=7.4 Hz, 3 H), 0.50 (t, J=7.4 Hz, 3 H); ¹³C NMR (100 MHz, DMSO-d₆) δ180.51, 146.95, 142.46, 141.92, 140.92, 128.68, 128.56, 127.72, 125.16, 120.92, 64.35, 63.80, 56.35, 55.59, 26.70, 23.96, 11.03, 8.61; IR (KBr) 3330.5, 2960.2, 2599.6, 2456.4, 2345.0, 1951.6, 1743.3, 1496.5, 1346.1, 1110.8, 1072.2, 852.4, 700.0, 586.3 cm⁻¹; HRMS (FAB⁺) for C₂₆H₃₁N₄O₂S [M+H]⁺ Calcd: 463.2168, Found: 463.2165
(7) 1-(4-Cyanophenyl)-3-[(1R,2R)-2-(pentan-3-ylamino)-1,2-diphenylethyl]thiourea (**1g**, Formula 2-7)
   69% yield; [α]_{D}²⁰=+55.5 (c=0.02, CH₂Cl₂); ¹H NMR (300 MHz, DMSO-d₆) δ10.3 (br s, 1 H), 8.54 (br s, 1 H), 7.84~7.72 (m, 4 H), 7.35~7.17 (m, 10 H), 5.54 (br s, 1 H), 4.14 (d, J=5.2 Hz, 1 H), 2.07 (br s, 1 H), 1.56 (br s, 1 H), 1.21 (m, 4H), 0.74 (t, J=7.4 Hz, 3 H), 0.49 (t, J=6.9 Hz, 3H); ¹³C NMR (100 MHz, DMSO-d₆) δ180.62, 144.83, 141.92, 141.05, 133.40, 128.67, 128.33, 127.68, 127.64, 127.51, 121.76, 119.76, 105.41, 64.41, 63.72, 60.43, 56.33, 26.74, 23.98, 21.42, 14.74, 11.02, 8.56; IR (KBr) 3317.0, 2960.2, 2360.4, 2225.5, 1949.7, 1739.5, 1508.1, 1315.2, 1176.4, 1072.2, 837.0, 700.0, 545.8 cm⁻¹; HRMS (FAB⁺) for C₂₇H₃₁N₄S [M+H]⁺ Calcd: 443.2269, Found: 443.2271
(8) 1-(4-Fluorophenyl)-3-[(1R,2R)-2-(pentan-3-ylamino)-1,2-diphenylethyl]thiourea (**1h**, Formula 2-8)
   84% yield; [α]_{D}²⁰=+17.9 (c=0.02, CH₂Cl₂); ¹H NMR (300 MHz, DMSO-d₆) δ9.83 (s, 1 H), 8.00 (d, J=6.7 Hz, 1 H), 7.48~7.43 (m, 2 H), 7.31~7.16 (m, 11 H), 5.46 (br s, 1 H), 4.09 (d, J=5.22 Hz, 1 H), 2.03 (br s , 1 H), 1.44 (br s, 1 H), 1.14 (m, 4 H), 0.70 (t, J=10.1, 3 H), 0.44 (t, J=7.0 Hz, 3 H); ¹³C NMR (100 MHz, DMSO-d₆) δ181.42, 161.03, 158.62, 141.90, 141.44, 135.97, 128.65, 128.51, 127.57, 127.42, 126.49, 116.13, 115.90, 64.39, 63.76, 56.03, 26.72, 24.02, 10.98, 8.39; IR (KBr) 3193.7, 2962.3, 1889.9, 1511.9, 1218.8, 848.6, 701.9, 555.42 cm⁻¹; HRMS (FAB⁺) for C₂₆H₃₁FN₃S[M+H]⁺ Calcd: 436.6172, Found: 436.2223. patern 436.5, 349.3, 266.4, 176.3, 106.1
(9) 1-((1R,2R)-2-(benzhydrylamino)-1,2-diphenylethyl)-3-(3,5-bis(trifluoromethyl)phenyl)thiourea (**1i**, Formula 2-9)
   95% yield; [α]_{D}²⁰=+0.39 (c=0.16, CH₂Cl₂); ¹H NMR (400 MHz, DMSO-d₆) δ7.82~7.09 (m, 23 H), 5.72 (s, 1 H), 3.98 (s, 1 H), 3.35 (s, 1 H), 2.47 (br, 1 H); ¹³C NMR (100 MHz, DMSO-d₆) δ181.06, 156.63, 153.35,143.36, 142.03, 141.31, 138.68, 129.48, 129.34, 126.90, 125.59, 123.65, 122.55, 122.14, 70.83, 65.14, 55.50; IR (KBr) 3239.9, 2964.2, 1471.5, 1278.6, 1135.9, 885.2, 700.1 cm⁻¹; HRMS (ESI⁺) for C₂₈H₃₀F₆N₃S [M+H]⁺ Calcd: 649.1986, Found: 649.1932
(10) 1-((1R,2R)-2-(benzhydrylamino)-1,2-diphenylethyl)-3-(4-(trifluoromethyl)phenyl)thiourea (**1j**, Formula 2-10)
   89% yield; [α]_{D}²⁰=+124 (c 0.10, CH₂Cl₂); ¹H NMR (300 MHz, DMSO-d₆) δ9.44 (br, 1H), 7.77~7.10 (m, 26H), 4.90 (s, 1H), 4.82 (s, 2H), 1.92 (s, 1H);IR(KBr) 3679.5, 3352.2, 2985.3, 1402.4, 1265.9, 1065.7, 726.8 cm⁻¹; HRMS(FAB⁺) for C₃₅H₃₀F₃N₃S [M+H]⁺ Calcd: 581.2113, Found: 581.2133
(11) 1-((1R,2R)-2-(3,5-dimethylbenzylamino)-1,2-diphenylethyl)-3-(3,5-dimethylphenyl)thiourea (**1k**, Formula 2-11)
   89% yield; [α]_{D}²⁰=+112 (c 0.13, CH₂Cl₂); ¹H NMR (500 MHz, DMSO-d₆) δ7.38 (t, 6H), 7.32 (d, 2H), 7.27 (d, 4H), 7.00 (s, 4H), 4.54 (s, 4H), 2.21 (s, 12H), 1.25 (br, 1H); ¹³C NMR (100 MHz, DMSO-d₆) δ167.08, 157.71, 156.95, 143.07, 138.05, 131.22, 129.00, 128.93, 127.09, 127.03, 123.43, 118.64, 112.63, 70.28, 68.05, 67.38, 21.63;IR(KBr) 3155.0, 2960.2, 2360.4, 1951.6, 1735.6, 1469.5, 1294.0, 1241.9, 1006.7, 837.0, 700.0, 572.8 cm⁻¹; HRMS(FAB⁺) for C₂₆H₃₀F₂N₃S[M+H]⁺ Calcd: 454.2129, Found: 454.2133.

### Example 2. Preparation of (S)-phenylpiracetam hydrochloride (Formula 1a)

A method for preparing (S)-phenylpiracetam hydrochloride (Formula 1a), a pharmaceutically acceptable salt of an (S)-type chiral gamma-lactam derivative according to one embodiment of the present disclosure, is shown by Scheme 2 below.

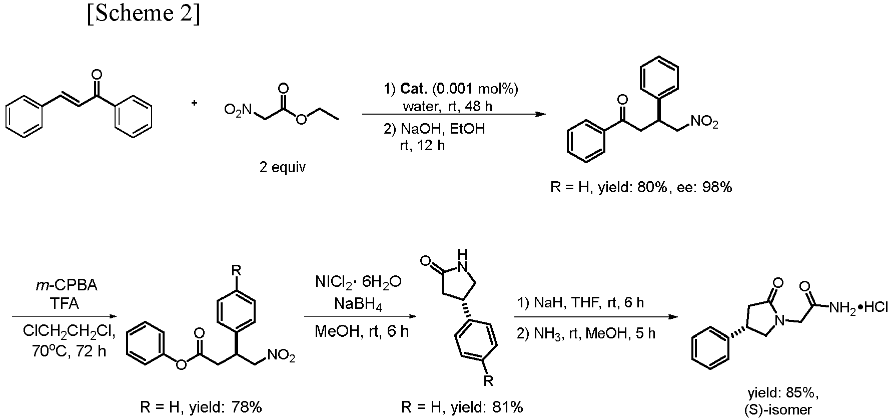

Using 1a of the chiral organocatalyst compounds prepared in Example 1, a Michael addition reaction was performed with nitro ethyl ester in the presence of water as a solvent to determine the reaction time and yield of α,β-unsaturated ketone compounds. Specifically, α,β-unsaturated ketone (1.0 eq), nitro ethyl ester (2.0 eq) and 0.1 to 0.001 mol% of chiral organocatalyst compound 1a were added to water (0.4 mL), and the reaction mixture was stirred at room temperature (rt). The reaction conversion was then monitored using TLC. After the reaction was completed, sodium hydroxide (1.0 eq) and ethanol were added thereto and the mixture was stirred at room temperature for 12 hours. Afterwards, the mixture was concentrated under reduced pressure to obtain the desired product. The product was purified by chromatography on a silica-gel column (hexane/ethylene acetate, 10:1).

### Example 2.1. (S)-4-Nitro-1,3-diphenyl-butan-1-one (3a, Formula 4a)

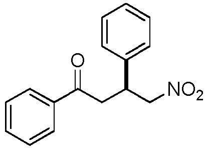

[α]_{D}²⁰ -18.5(*c* 1.0, CHCl₃); ¹H NMR(500 MHz, CDCl₃) δ 7.91~7.92(m, 2H), 7.59~7.26(m, 8H), 4.85~4.81(dd, 1H), 4.71~4.67(dd, 1H), 4.26~4.20(m, 1H), 3.51~3.46(dd, 1H), 3.45~3.40(dd, 1H) ppm; ¹³C NMR(125 MHz, CDCl₃) δ 196.87, 139.15, 136.39, 133.60, 129.09, 128.77, 128.04, 127.90, 127.48, 79.58, 41.54, 39.30 ppm; IR(KBr) 3058, 3029, 2920, 1687, 1544, 1440, 1367, 1268, 1224, 1084, 988, 764, 703, 623, 559 cm⁻¹; LRMS(ESI⁺) for C₁₆H₁₅NO₃ [M+Na]⁺ Calcd: 292.1, Found: 292.1; HPLC [Chiralcel AD-H, hexane/2-propanol = 90/10, flow rate = 1.0 mL/min, λ = 254 nm, retention times:(major) 12.8 min,(minor) 17.4 min]; *R_{f}*(SiO₂, EtOAc/n-hexane = 1/5) = 0.40

### Example 2.2. (S)-Phenyl-4-nitrobutanoate derivative (Formula 5a)

### (1) (S)-Phenyl 4-nitro-3-phenylbutanoate (4a, Formula 5a-1)

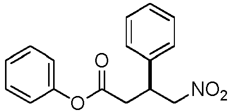

[α]_{D}²⁰ +7.7(*c* 1.0, CHCl₃); ¹H NMR(500 MHz, CDCl₃) δ 7.38~7.16(m, 8H), 6.87~6.85(m, 2H), 4.75~4.71(dd, *J* = 11.6, 6.6 Hz, 1H), 4.68~4.64(dd, *J* = 11.6, 6.4 Hz, 1H), 4.10~4.04(m, 1H), 3.04~3.0(dd, *J* = 13.7, 4.6 Hz, 1H), 2.99~2.94(dd, *J* = 13.7, 5.6 Hz, 1H) ppm; ¹³C NMR(125 MHz, CDCl₃) δ 169.33, 150.33, 137.96, 129.50, 129.22, 128.29, 127.55, 126.12, 121.39, 79.38, 40.38, 37.87 ppm; LRMS(ESI⁺) for C₁₆H₁₅NO₄ [M+Na]⁺ Calcd: 308.1, Found: 308.1; *R_{f}*(SiO₂, EtOAc/n-hexane = 1/5) = 0.40

### (2) (S)-Phenyl 3-(4-chlorophenyl)-4-nitrobutanoate (4b, Formula 5a-2)

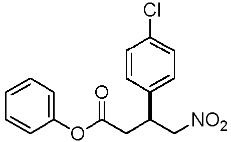

[α]_{D}²⁰ +20.8(*c* 1.0, CH₂Cl₂); ¹H NMR(500 MHz, CDCl₃) δ 7.35~7.22(m, 7H), 6.92~6.90(m, 2H), 4.79~4.75(dd, *J* = 12.7, 7.3 Hz, 1H), 4.71~4.67(dd, *J* = 12.7, 7.9 Hz, 1H), 4.11~4.06(m, 1H), 3.08~3.03(dd, *J* = 14.7, 5.3 Hz, 1H), 3.01~2.96(dd, *J* = 14.7, 6.5 Hz, 1H) ppm; ¹³C NMR(125 MHz, CDCl₃) δ 169.01, 150.17, 136.37,134.23, 129.53, 129.43, 128.88, 126.20, 121.26, 79.08, 39.70, 37.67 ppm; LRMS(ESI⁺) for C₁₆H₁₄ClNO₄ [M+Na]⁺ Calcd: 342.1, Found: 342.1; *R_{f}*(SiO₂, EtOAc/*n*-hexane = 1/5) = 0.31

### Example 2.3. (S)-4-Phenylpyrrolidin-2-one (4c, Formula 6a)

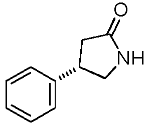

[*α*]_{D}²⁰+33.3(*c* 1.0, MeOH);
¹H NMR(500 MHz, CDCl₃) δ 7.37~7.33(m, 2H), 7.29~7.27(m, 2H), 7.26~7.25(m, 1H), 5.92(br s, 1H), 3.81~3.77(m, 1H), 3.71(q, *J* = 8.0 Hz, 1H), 3.45~3.41(dd, *J =* 9.4, 2.0 Hz, 1H), 2.77~2.72(dd, *J* = 16.8, 8.7 Hz, 1H), 2.54~2.49(dd, *J* = 17.0, 8.5 Hz, 1H) ppm; ¹³C NMR(125 MHz, CDCl₃) δ 177.93, 142.14, 128.88, 127.13, 126.79, 49.60, 40.31, 38.02 ppm; LRMS(ESI⁺) for C₁₀H₁₁NO[M+H]⁺Calcd: 162.1, Found: 162.2; HPLC [Chiralcel AD-H, hexane/2-propanol = 90/10, flow rate =1.0 mL/min, λ = 254 nm, retention times:(minor) 15.2 min,(major) 19.4 min]; *R_{f}*(SiO₂, EtOAc) = 0.30

### Example 2.4. (S)-2-(2-oxo-4-phenylpyrrolidin-1-yl)acetamide hydrochloride (5a, Formula 1a)

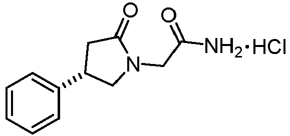

[α]_{D}²⁰ = -7.3 (c = 1.0, MeOH);
A solution of 4-phenyl-2-pyrrolidinone (1.0 eq) in 1,4-dioxane (30 ml) was added to a suspension of sodium hydride (1.1 eq) in 1,4-dioxane (30 ml). The mixture was heated at 90°C for 30 minutes and then cooled to room temperature. Ethyl bromoacetate (1.1 eq) was added thereto, and the reaction mixture was refluxed at 120°C for 6 hours. The obtained mixture was concentrated under reduced pressure. The residue was purified by column chromatography on a silica gel column using a 1:1 ethyl acetate-hexane mixture to give N-ethoxy carbonyl methyl-4-phenyl-2-pyrrolidinone. A solution of N-ethoxy carbonyl methyl-4-phenyl-2-pyrrolidinone (250 mg, 1.01 mM) in methanol (30 ml) was saturated with a stream of gaseous ammonia for 5 h. The reaction mixture was concentrated under reduced pressure, and the residue was purified by column chromatography on silica gel using a 1:1 ethyl acetate-hexane mixture and concentrated under reduced pressure. The concentrate was added to 6N HCl, and this mixture was stirred until the concentrate was dissolved. Then, the reaction mixture was concentrated under reduced pressure again to obtain N-carbamoyl methyl-4-phenyl-2-pyrrolidinone chloride (85%).
¹H NMR spectrum(DMSO-*d*₆), δ, ppm: 2.41 d.d(1H, 3-H), 2.72 d.d(1H, 3-H), 3.42 m(1H, 5-H), 3.59 m(1H, 4-H), 3.79 m(1H, 5-H), 4.00 d.d(2H, NCH₂CO); 7.24-7.36 m(5H, Ph). ¹³C NMR spectrum(DMSO-*d*6), δC, ppm: 37.01, 38.48, 44.04, 54.55, 127.16, 127.35, 129.08, 143.35, 170.64, 173.84 pp; LRMS(ESI⁺) for C₁₀H₁₄ClNO₂ [M]⁺ Calcd: 254, Found: 254;

### Example 3. Preparation of (R)-phenylpiracetam hydrochloride (Formula 1b)

A method for preparing (R)-phenylpiracetam hydrochloride (Formula 1b) and (R)-phenylpiracetam hydrazide hydrochloride (Formula 1d), which are pharmaceutically acceptable salts of (R)-type chiral gamma-lactam derivatives according to one embodiment of the present disclosure, is shown by Scheme 3 below. 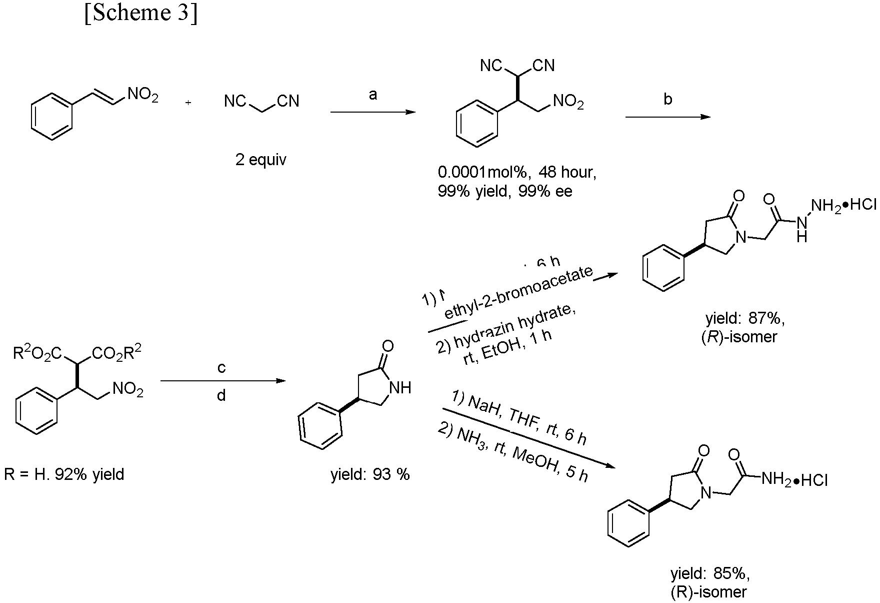
a) Catalyst compound **1a** (0.0001 mol %), water, room temperature, 48h; b) Magnesium monoperoxyphthalate hexahydrate (MMPP), Li₂CO₃, MeOH, 0°C, 1 hour; c) NiCl₂·6H₂O/NaBH₄, MeOH, room temperature, 6h; d) (1) NaOH, EtOH, room temperature, 0.5h; (2) Toluene, reflux, 0.5h.

### Example 3.1. (R)-malonate derivative (Formula 5b)

A method for preparing an intermediate (R)-malonate derivative for preparing a pharmaceutically acceptable salt of the (R)-type chiral gamma-lactam derivative is shown by Scheme 4 below.

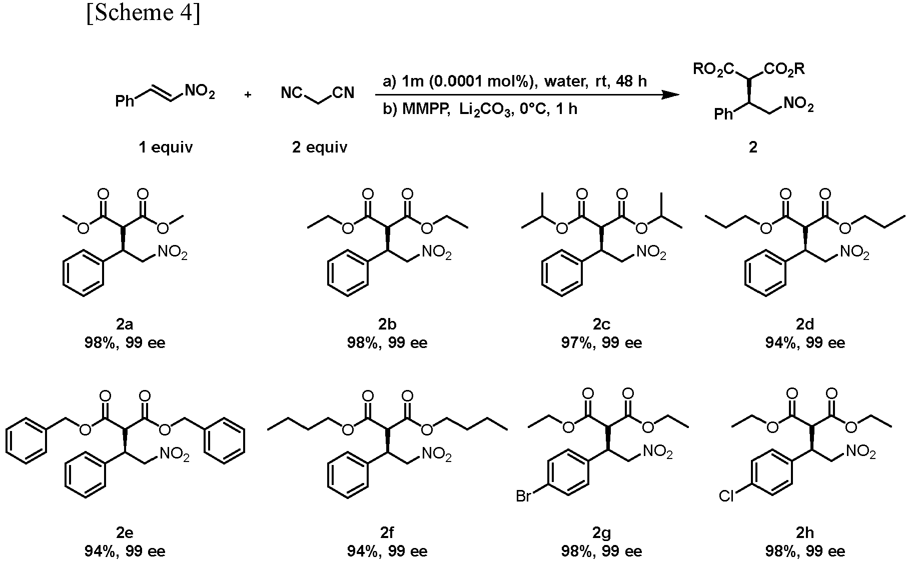

In the presence of (0.001 mol %) malonitrile (2.0 eq) and the chiral organocatalyst compound **1a** prepared in Example 1, α,β-Unsaturated nitro compound was mixed with trans-β-nitrostyrene (1.0 eq) and malononitrile (2.0 eq) using water (0.4 mL) as a solvent, and the reaction mixture was stirred at room temperature. The reaction conversion was monitored by TLC, and after the reaction was completed, 6 N HCl was added to the reaction mixture and the mixture was heated at 65°C for 2 hours. After the reaction mixture was cooled to room temperature, dialkyl carbonate (1.5 eq) was added thereto and the solution was heated while being stirred at 100°C for 3 hours. The homogeneous reaction mixture was then cooled to room temperature, poured into a 10% aq. NaHCO₃ solution, and ethyl acetate (0.2 ml) was added to the reaction mixture. The solution was washed twice with water (2 × 1.0 mL), dried over magnesium sulfate, and concentrated to obtain the desired product. The product was then purified by chromatography on a silica gel column (hexane/methylene chloride 2:1).

Under an argon atmosphere, to a suspension of the Michael addition reaction product obtained above (1.0 eq, >99% ee) and NiCl₂·6H₂O (1.0 eq) in MeOH (8.0 mL), NaBH₄ (10 eq) was added at 0°C. The reaction mixture was stirred at room temperature for 7.5 hours, and then the reaction mixture was quenched with NH₄Cl, and diluted with CHCl₃. The organic layer was separated, filtered over MgSO₄ and concentrated in a vacuum. The residue was purified by silica gel chromatography (MeOH/CHCl₃ = 1:20 as a solvent) to obtain the desired product as a colorless powder.

### (1) (R)-Dimethyl 2-(2-nitro-1-phenylethyl)malonate (2a, Formula 5b-1)

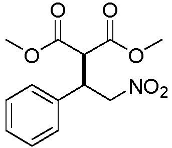

[α]_{D}²⁰ = -1.98(*c* 1.33, CH₂Cl₂); ¹H NMR(300 MHz, CDCl₃) δ 7.40~7.15(m, 5H), 5.15~5.03(m, 1H), 4.93(dd, *J* = 4.5, 12.8 Hz, 1H), 4.88~4.76(m, 2H), 4.20(td, *J* = 4.5, 9.5 Hz, 1H), 3.76(d, *J =* 9.5 Hz, 1H), 1.24(d, *J=* 6.1 Hz, 3H), 1.07(d, *J =* 6.4 Hz, 3H), 1.01(d, *J* = 6.4 Hz, 3H) ppm; ¹³C NMR(100 MHz, CDCl₃) δ 167.1, 166.4, 136.3, 128.9, 128.3, 128.2, 77.9, 69.9, 69.5, 55.1, 42.9, 21.5, 21.4, 21.19, 21.17 ppm; IR(KBr) 3030, 2985,1727, 1557 cm⁻¹; HRMS(ESI) for C₁₃H₁₆N₁O₆[M+H]⁺ Calcd: 282.09721, Found: 282.09726; HPLC [Chiralcel AD-H, hexane/2-propanol = 95/5, 1.0 mL/min, λ = 254 nm, retention times:(major) 23.3 min,(minor) 38.0 min].

### (2) (R)-Diethyl 2-(2-nitro-1-phenylethyl)malonate (2b, Formula 5b-2)

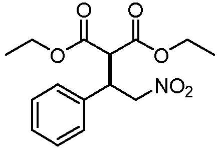

[α]_{D}²⁰ = -4.61(*c* 0.23, CH₂Cl₂); ¹H NMR(300 MHz, CDCl₃) δ 7.30~7.20(m, 5H), 4.93(dd, *J* = 4.6, 13.1 Hz, 1H), 4.86(dd, *J* = 9.2, 13.1 Hz, 1H), 4.24~4.17(m, 3H), 3.98~3.97(q, 2H), 3.81~3.79(d, 1H), 1.25(t, 3H), 1.03(t, 3H) ppm; ¹³C NMR(100 MHz, CDCl₃) δ 167.4, 166.8, 136.2, 128.8, 128.2, 77.4, 77.0, 76.7 62.1, 61.8, 54.9, 42.9, 13.9, 13.7 ppm; IR(KBr) 2989, 2938, 1731, 1557 cm⁻¹; HRMS(ESI) for C₁₅H₂₀N₁O₆[M+H]⁺ Calcd: 310.12851, Found: 310.12936; HPLC [Chiralcel AD-H, hexane/ethanol = 90/10, 1.0 mL/min, λ = 254 nm, retention times:(major) 11.5 min,(minor) 15.3 min].

### (3) (R)-Diisopropyl 2-(2-nitro-1-phenylethyl)malonate (2c, Formula 5b-3)

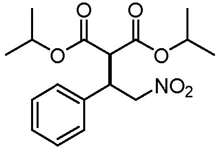

[α]_{D}²⁰ = -1.24(*c* 1.00, CH₂Cl₂); ¹H NMR(300 MHz, CDCl₃) δ 7.32~7.22(m, 5H), 5.10(dd, *J* = 5.0,13.1 Hz, 1H), 4.91~4.979(m, 3H), 4.21~4.19(m, 1H), 1.25(d, *J* = 2.0 Hz, 6H), 1.07(dd, *J* = 2.0,2.0 Hz, 6H) ppm; ¹³C NMR(100 MHz, CDCl₃) δ 167.27, 166.54, 136.47, 129.07, 128.34, 127.9, 78.15, 70.15, 69.75, 55.35, 43.14, 21.80, 21.67, 21.48 ppm; IR(KBr) 3029, 2956, 1737, 1558 cm⁻¹; HRMS(ESI) for C₁₇H₂₄N₁O₆[M+H]⁺ Calcd: 338.15981 Found: 338.16336; HPLC [Chiralcel AD-H, hexane/2-propanol = 95/5, 1.0 mL/min, λ = 254 nm, retention times:(major) 14.8 min,(minor) 34.4 min].

### (4) (R)-Dipropyl 2-(2-nitro-1-phenylethyl)malonate (2d, Formula 5b-4)

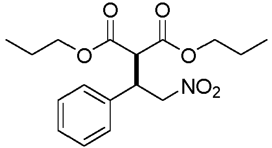

[α]_{D}²⁰ = -1.73(*c* 0.10, CH₂Cl₂); ¹H NMR(300 MHz, CDCl₃) δ 7.31~7.22(m, 5H), 4.92~4.87(t, *J* = 5.0,9.5 Hz, 2H), 4.24(m, 1H), 4.15~4.09(m, 2H), 3.92~3.83(dd,s, *J* =6.6 9.7 Hz, 3H), 1.68~1.61(m, 2H), 1.49~1.42(m, 2H), 0.93~0.88(t, *J* = 7.4,7.4 Hz, 3H), 0.82~0.77(t, *J* = 7.4,7.4 Hz, 3H) ppm; ¹³C NMR(100 MHz, CDCl₃) δ 167.79, 167.17, 136.46, 129.14, 128.52, 128.17, 77.85, 67.86, 67.65, 55.17, 43.16, 21.97, 21.81, 10.48 ppm; IR(KBr) 3029, 2956, 1737, 1558 cm⁻¹; HRMS(ESI) for C₁₇H₂₄N₁O₆[M+H]⁺ Calcd: 338.15981 Found: 338.16336; HPLC [Chiralcel AD-H, hexane/2-propanol = 95/5, 1.0 mL/min, λ = 254 nm, retention times:(major) 18.4 min,(minor) 38.9 min].

### (5) (R)-Benzyl-2-carbobenzyloxy-4-nitro-3-phenylbutyrate (2e, Formula 5b-5)

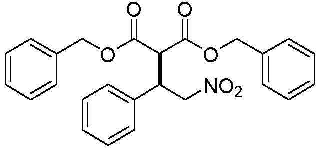

[α]_{D}²⁰ = -3.25(c 0.10, CH₂Cl₂); ¹H NMR(400 MHz, CDCl₃) δ 7.33-7.25(m, 10H), 7.17-7.07(m, 5H), 5.16(d, 1H, J = 12.2 Hz), 5.18(d, 1H, JAB = 12.2 Hz), 4.93(S, 1H), 4.84-4.82(m, 2H), 4.28~4.22(q, 1H), 3.94(d, 1H, 9.3 Hz); 13C NMR(100 MHz, CDCl₃) δ 167.39, 166.78, 136.14, 134.85, 129.25, 128.90, 128.15, 77.63, 68.04, 67.86, 55.14, 43.16 ppm; IR(KBr) 3068, 3036, 2963, 1736, 1558, 1498, 1456, 1378, 1326, 1286, 1217, 1156, 1003, 975, 908, 562 cm⁻¹; HRMS(EI) for C₂₅H₂₃N₁O_{6[}M+H]⁺ Calcd: 433.1525 Found: 433.1525; HPLC [Chiralcel AD-H, hexane/2-propanol = 70/30, 1.0 mL/min, λ = 254 nm, retention times:(major) 26.0 min,(minor) 24.1 min].

### (6) (R)-Dibutyl 2-(2-nitro-1-phenylethyl)malonate (2f, Formula 5b-6)

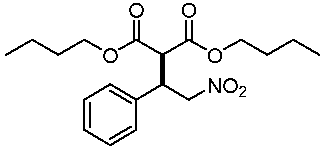

[α]_{D}²⁰ = -2.55(*c* 0.10, CH₂Cl₂); ¹H NMR(400 MHz, CDCl₃) δ 7.31~7.22(m, 5H), 4.92~4.87(t, *J* = 5.0,9.5 Hz, 2H), 4.24(m, 1H), 4.15~4.09(m, 2H), 3.92~3.83(dd,s, *J* =6.6 9.7 Hz, 3H), 1.68~1.61(m, 2H), 1.49~1.42(m, 2H), 0.93~0.88(t, *J* = 7.4,7.4 Hz, 3H), 0.82~0.77(t, *J* = 7.4,7.4 Hz, 3H) ppm; ¹³C NMR(100 MHz, CDCl₃) δ 167.79, 167.17, 136.46, 129.14, 128.52, 128.17, 77.85, 67.86, 67.65, 55.17, 43.16, 21.97, 21.81, 10.48 ppm; HRMS(EI) for C₁₇H₂₄N₁O₆[M+H]⁺ Calcd: 338.15981 Found: 338.16336; HPLC [Chiralcel AD-H, hexane/2-propanol = 95/5, 1.0 mL/min, λ = 254 nm, retention times:(major) 18.4 min,(minor) 38.9 min].

### (7) (R)-Diethyl 2-[1-(4-bromophenyl)-2-nitroethyl]malonate (2g, Formula 5b-7)

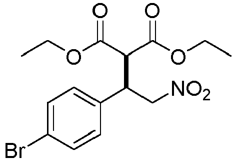

[α]_{D}²⁰ = -3.56(*c* 2.33, CH₂Cl₂); ¹H NMR(300 MHz, CDCl₃) δ 7.44~7.42(d, *J* = 8.5 Hz, 2H), 7.13~7.11(d, *J* = 8.2 Hz, 2H), 4.88~4.81(m, 2H), 4.22~4.16 (m, 3H), 4.04~3.97(q, *J* = 7.1,6.9 Hz, 2H), 3.78~3.75(d, J = 9.4 Hz, 1H), 1.26~1.21(t, *J* = 7.2,7.1 Hz, 3H), 1.08∼1.03(t, *J* = 7.1,7.1 Hz, 3H) ppm; ¹³C NMR(100 MHz, CDCl₃) δ 167.42, 166.83, 135.52, 132.29, 130.00, 122.62, 77.55, 62.50, 62.26, 54.86, 42.60, 14.17, 13.99 ppm; IR(KBr) 2983, 2950, 1732, 1556, 1490, 1445 cm⁻¹ HRMS(ESI) for C₁₅H₁₉N₁O₆Br[M+H]⁺ Calcd: 388.03903 Found: 388.04495; HPLC [Chiralcel AD-H, hexane/ethanol = 95/5, 1.0 mL/min, λ = 254 nm, retention times:(major) 35.9 min,(minor) 44.4 min].

### (8) (R)-Diethyl 2-(1-(4-chlorophenyl)-2-nitroethyl)malonate (2h, Formula 5b-8)

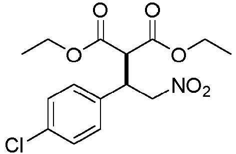

[α]_{D}²⁰ = -0.24(c 0.43, CH₂Cl₂); ¹H NMR(300 MHz, CDCl₃) δ 7.29~7.17(dd, *J* = 20.6,8.2 Hz, 4H), 4.88~4.81(m, 2H), 4.23~4.16(m, 3H), 4.04~3.97(q, *J* = 7.1,7.1 Hz, 2H), 3.78~3.75(d, *J* = 9.3 Hz, 1H), 1.26~1.21(t, *J* = 7.1,7.2 Hz, 3H), 1.08~1.03(t, J= 7.2,6.8 Hz, 3H) ppm; ¹³C NMR(100 MHz, CDCl₃) δ 167.44, 166.83, 134.98, 134.46, 129.69, 129.32, 77.63, 62.49, 62.23, 54.92, 42.55, 14.15, 13.97 ppm; IR(KBr) 2984, 1733, 1557, 1478, 1445, 1371 cm⁻¹ HRMS(ESI) for C₁₅H₁₉N₁O₆Cl[M+H]⁺ Calcd: 344.08954 Found: 344.09119; HPLC [Chiralcel AD-H, hexane/ethanol = 90/10, 1.0 mL/min, λ = 254 nm, retention times:(major) 17.9 min,(minor) 24.1 min].

### Example 3.2. (R)-4-Phenylpyrrolidin-2-one (4d, Formula 6b)

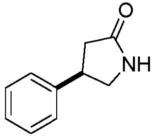

[α]_{D}²⁰ -32.1(c 1.0, MeOH);
¹H NMR(500 MHz, CDCl₃) δ 7.37~7.33(m, 2H), 7.29~7.27(m, 2H), 7.26~7.25(m, 1H), 5.92(br s, 1H), 3.81~3.77(m, 1H), 3.71(q, *J* = 8.0 Hz, 1H), 3.45~3.41(dd, *J =* 9.4, 2.0 Hz, 1H), 2.77~2.72(dd, *J* = 16.8, 8.7 Hz, 1H), 2.54~2.49(dd, *J* = 17.0, 8.5 Hz, 1H) ppm; ¹³C NMR(125 MHz, CDCl₃) δ 177.93, 142.14, 128.88, 127.13, 126.79, 49.60, 40.31, 38.02 ppm; LRMS(ESI⁺) for C₁₀H₁₁NO[M+H]⁺Calcd: 162.1, Found: 162.2; HPLC [Chiralcel AD-H, hexane/2-propanol = 90/10, flow rate =1.0 mL/min, λ = 254 nm, retention times:(minor) 15.2 min,(major) 19.4 min]; *R_{f}*(SiO₂, EtOAc) = 0.30

### Example 3.3. (R)-2-(2-oxo-4-phenylpyrrolidin-1-yl)acetamide hydrochloride (5b, Formula 1b)

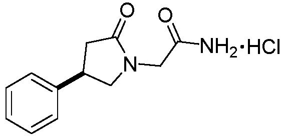

[α]_{D}²⁰ = +7.4 (c = 1.0, MeOH);
N-carbamoyl methyl-4-phenyl-2-pyrrolidinone chloride (85%) was obtained by the same synthetic method as in Example 2.4.
¹H NMR spectrum(DMSO-*d*₆), δ, ppm: 2.41 d.d(1H, 3-H), 2.72 d.d(1H, 3-H), 3.42 m(1H, 5-H), 3.59 m(1H, 4-H), 3.79 m(1H, 5-H), 4.00 d.d(2H, NCH₂CO); 7.24-7.36 m(5H, Ph). ¹³C NMR spectrum(DMSO-d6), δC, ppm: 37.01, 38.48, 44.04, 54.55, 127.16, 127.35, 129.08, 143.35, 170.64, 173.84 pp; LRMS(ESI⁺) for C₁₀H₁₄ClNO₂ [M]⁺ Calcd: 254, Found: 254;

### Example 4. Preparation of (R)-phenylpiracetam hydrazide hydrochloride

### (Formula 1d)

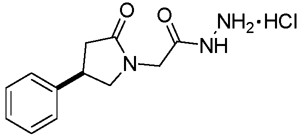

A method for preparing (R)-phenylpiracetam hydrazide hydrochloride (Formula 1d), a pharmaceutically acceptable salt of an (R)-type chiral gamma-lactam derivative according to one embodiment of the present disclosure, is shown by Scheme 3. (R)-4-Phenylpyrrolidin-2-one (4d, Formula 6b) was obtained in the same manner as in Example 3.

Then, a solution of 4-phenyl-2-pyrrolidinone (1.0 eq) in 1,4-dioxane (30 ml) was added to a suspension of sodium hydride (1.1 eq) in 1,4-dioxane (30 ml). The mixture was heated at 90°C for 30 minutes and then cooled to room temperature. Ethyl bromoacetate (1.1 eq) was added thereto, and the reaction mixture was refluxed at 120°C for 6 hours. The obtained mixture was concentrated under reduced pressure. The residue was purified by column chromatography on a silica gel column using a 1:1 ethyl acetate-hexane mixture to obtain N-ethoxy carbonyl methyl-4-phenyl-2-pyrrolidinone. A solution of N-ethoxy carbonyl methyl-4-phenyl-2-pyrrolidinone (250 mg, 1.01 mM) in methanol (30 ml) was saturated with a stream of gaseous ammonia for 5 h. The reaction mixture was concentrated under reduced pressure, and the residue was purified by column chromatography on a silica gel column using a 1:1 ethyl acetate-hexane mixture and concentrated under reduced pressure. The concentrate and hydrazine hydrate (1.1 eq) were dissolved in ethanol (30 ml), and the mixture was stirred at room temperature for 1 hour. Then, the mixture was concentrated. The concentrate was added to 6N HCl, and this mixture was stirred until the concentrate was dissolved. Then, the reaction mixture was concentrated under reduced pressure again to obtain N-carbamoyl methyl-4-phenyl-2-pyrrolidinone hydrazine chloride (87%).
¹H NMR spectrum(DMSO-*d*₆), δ, ppm: 2.51 d.d(1H, 3-H), 2.70 d.d(1H, 3-H), 3.43 m(1H, 5-H), 3.62 m(1H, 4-H), 3.77 m(1H, 5-H), 3.87 d.d(2H, NCH₂CO), 4.26 br.s(2H, NH-NH₂), 7.24-7.34 m(5H, Ph), 9.19 br.s(1H, CONH). ¹³C NMR spectrum(DMSO-*d*₆), δC, ppm: 36.47, 37.99, 43.54, 54.07, 126.68, 126.90, 128.61, 142.88, 170.20, 173.30 pp; LRMS(ESI⁺) for C₁₂H₁₆ClNO₂ [M+H]⁺ Calcd: 270, Found: 270;

### Experimental Example 1. In vitro efficacy analysis of chiral gamma-lactam derivatives of the present disclosure or pharmaceutically acceptable salts thereof

The efficacy of the chiral gamma-lactam derivative of the present disclosure or a pharmaceutically acceptable salt thereof in controlling muscular disease, mental disease, and degenerative neurological disease was analyzed using a human kidney, skeletal muscle, and a neuronal cell line. Through chirality modulation and derivatization, it was confirmed that the chiral gamma-lactam derivative of the present disclosure or a pharmaceutically acceptable salt thereof is excellent in effectiveness in the calcium excretion control mechanism; and through cytotoxicity analysis, it was confirmed that the derivative or a pharmaceutically acceptable salt thereof is not different from natural amino acids. Through investigating the intracellular mode of action, it was confirmed that the derivative of the present disclosure or a pharmaceutically acceptable salt thereof inhibits MAO-B as a target for D1-mGlu5 and regulates calcium channels. In addition, the inventors confirmed that myostatin regulatory factor (TNF-beta), a marker of sarcopenia, was controlled, and that protein expression was inhibited.

### Experimental Example 1.1. Measuring intracellular Ca²⁺ concentration using confocal laser scanning microscopy (CLSM)

To detect intracellular Ca²⁺ levels, the HEK293T cell line, human renal epithelial cells, were seeded in 35 mm diameter confocal dishes 24 hours before the experiment and then were cultured to 40-60% confluency. The cells were loaded with 5 mM fluorescent radioactive calcium indicator Fluo-3-acetoxymethyl (Fluo-3-AM; Invitrogen) for 30 min at 37°C. The concentration of Ca²⁺ was determined using CLSM (Zeiss LSM 700 Meta; Zeiss, Oberkochen, Germany). The culture plates were washed with culture medium, and then the culture plates were placed on a temperature-controlled microscope stage and were observed at 200X magnification. The excitation and emission wavelengths for signal detection were 488 nm and 515 nm, respectively. Analysis of intracellular calcium intensity was performed using Zen software (Carl Zeiss).

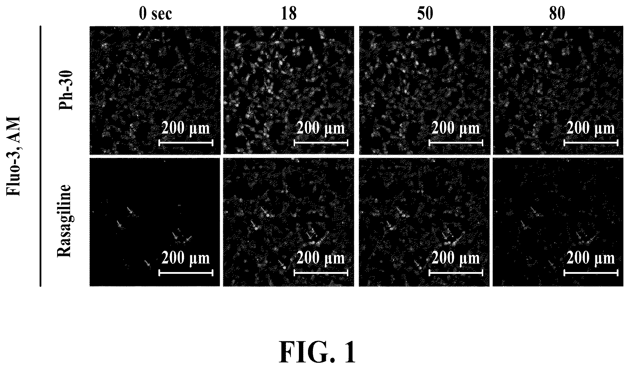

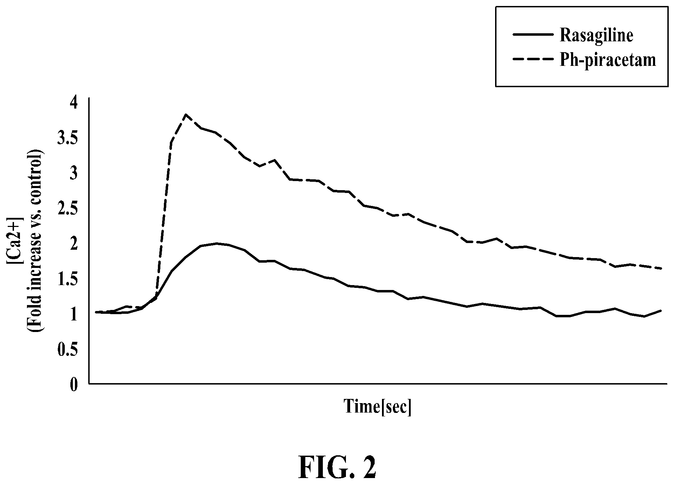

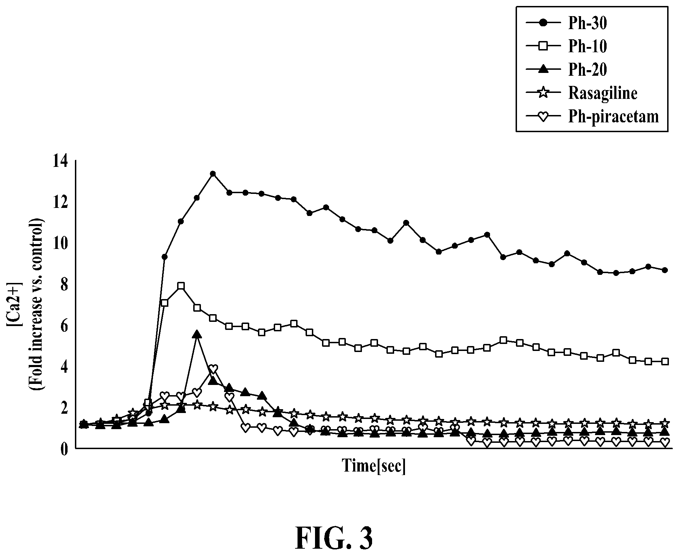

The results show that the intensity of intracellular calcium was highest at 18 seconds and then gradually decreased as calcium was released (FIG. 1). Compared to the control, rasagiline, and the comparator, (R)-phenylpiracetam (Ph-piracetam), the Ca²⁺ release performance of pharmaceutically acceptable salts of chiral gamma-lactam derivatives according to one embodiment of the present disclosure, Ph-10 (Formula 1d), Ph-20 (Formula 1a), and Ph-30 (Formula 1b) was far superior (FIGS. 2 and 3). In particular, in the case of (R)-type Ph-30, compared to (S)-type Ph-20 the results show a calcium release performance difference of about 2 times or more, and in the case of hydrochloride substitution the results show a calcium release performance difference of about 1.5 to 3 times or more. This difference is thought to be a result of the enhanced molecular polarity properties due to increased polarizability and dipole moment for Ph-10, 20, and 30 compounds compared to phenylpiracetam, which promotes non-bonded interactions in the drug-receptor complex.

### Experimental Example 1.2. Human skeletal myoblast (HSkM) differentiation induction test

For the human skeletal myoblast (HSkM) differentiation induction test, human skeletal myoblasts (3 x 10⁵ cells/well) were prepared in 24-well plates. Then, these cells were treated with the test substances and incubated in a 37°C, CO₂ incubator for 48 hours. After 24 and 48 hours of incubation, the cell culture supernatant was collected into an E-tube and stored in a deep freezer. For ELISA analysis, the supernatant was thawed and prepared. ELISA analysis was performed based on the test method presented in the ELISA kit.

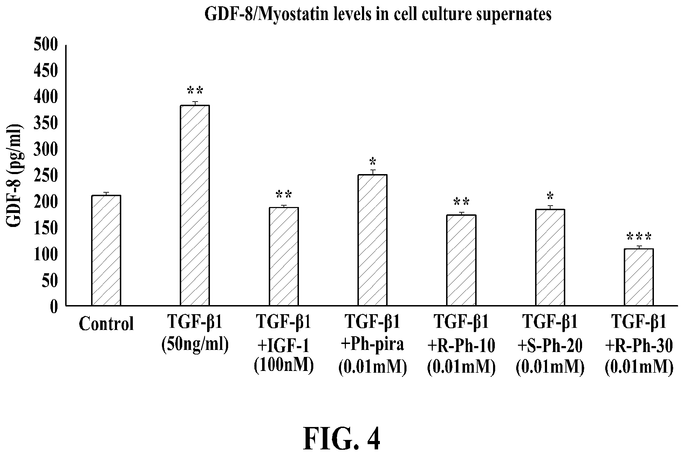

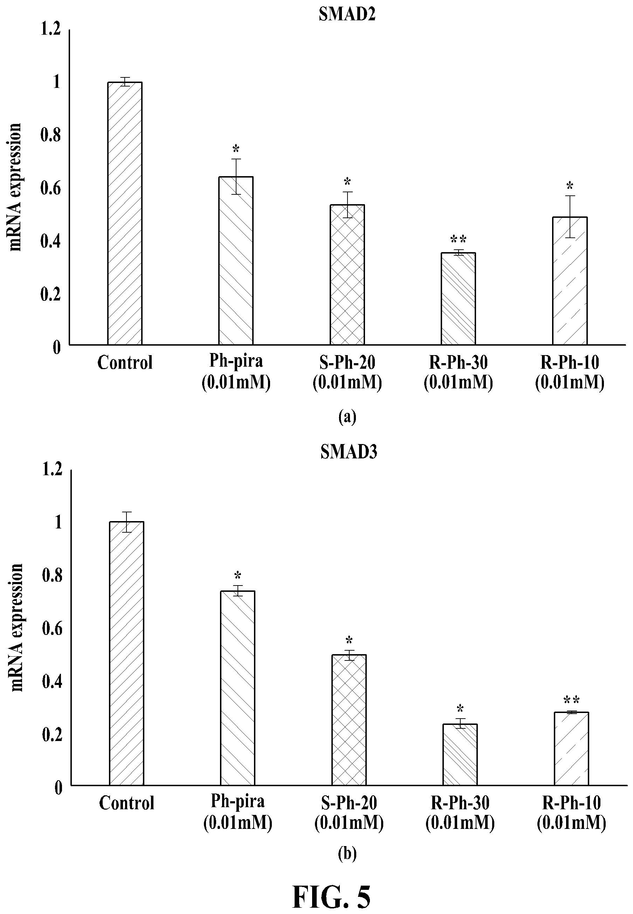

The results showed that the Ph-30 compound reduced GDF-8/Myostatin level the most, while the Ph-10 and 20 compounds each performed equally or better compared to the compounds of the IGF-1 and (R)-phenylpiracetam (Ph-pira) treatment groups (FIG. 4). In addition, it was confirmed that the Ph-30 compound lowered the SMAD2 and SMAD3 levels the most, while the Ph-10 and 20 compounds also showed effective performance compared to the compound of the (R)-phenylpiracetam treatment group (FIG. 5).

### Experimental Example 1.3. Confirming the changes in mRNA expression related to neuroinflammation and apoptosis (cell death)

After inducing an inflammatory response by treating a human neuroblastoma SH-SY5Y cell line with 2.5 mM MPTP (1-methyl-4-phenyl-1,2,3,6-tetrahydropyridine), the mRNA expression of SYP (synaptophysin), GAP-43 (Growth-associated protein-43), MAO-B (Monoamine oxidase-B), and iNOS (inducible nitric oxide synthase) was confirmed by real-time PCR.

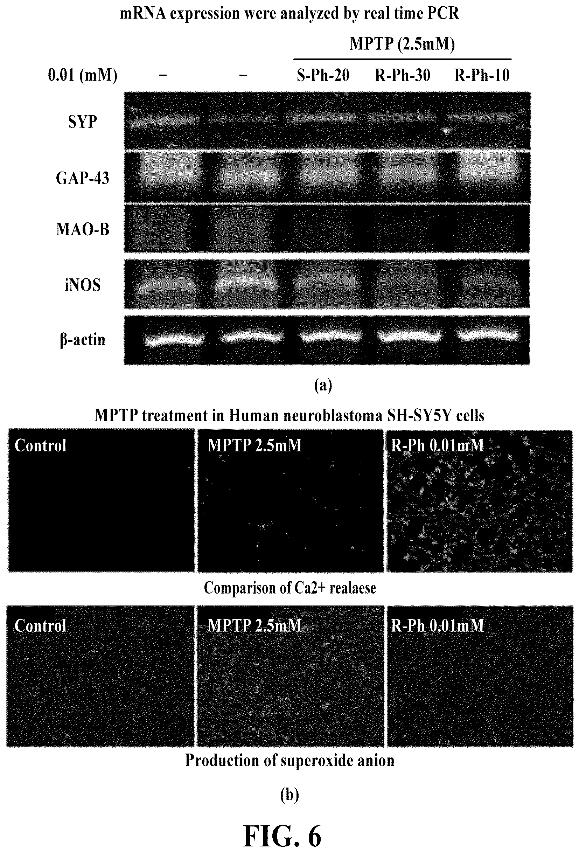

The results showed that the mRNA expression of SYP was significantly increased when the treatment group was treated with the chiral gamma lactam derivative of the present disclosure or a pharmaceutically acceptable salt thereof (Ph-10, 20, 30) compared to when the treatment group was treated with MPTP alone; the mRNA expression of GAP-43 was significantly increased in the Ph-10 compound treatment group compared to the treatment group treated with MPTP alone; and the mRNA expression of iNOS and MAO-B was significantly reduced in the Ph-10 and 30 compound treatment groups compared to the treatment group treated with MPTP alone (FIG. 6 (a)). In particular, in the case of the Ph-30 compound, it was confirmed through fluorescence microscopy that Ca²⁺ was significantly increased and that superoxide anions were significantly reduced after the MPTP treatment (FIG. 6 (b)).

### Experimental Example 2. In vivo efficacy analysis of chiral gamma-lactam derivatives of the present disclosure or pharmaceutically acceptable salts thereof

Six-month-old normal mice were acclimated for one week, trained for behavioral testing, and then randomly divided into groups. To construct a Parkinson's disease mouse MPTP model, mice were treated with the chiral gamma-lactam derivative of the present disclosure or a pharmaceutically acceptable salt thereof (dissolved in 0.9% saline, intraperitoneally injected (ip)) for 3 days, and then were treated with MPTP (dissolved in 0.9% saline, ip) for 5 consecutive days. The MPTP treatment caused damage to the cerebellum, resulting in decreased muscle tone and impaired motor control. For the control mice group, the same volume of saline solution (ip) was provided. Two days after the last injection, the efficacy was confirmed by an open field test (OFT), forced swim test (FST), rotarod test, and pilocarpine-induced seizure evaluation.

In addition, blood analysis confirmed a decrease in the amount of Myostatin in the blood, a marker related to sarcopenia, and confirmed that there was no toxicity effect on liver function, kidney function, or myocardial function. The mechanism of action was confirmed through skeletal muscle tissue analysis, calcium levels, motility, and blood tests.

### Experimental Example 2.1. Open Field Test (OFT)

To measure general motor activity levels and behavioral anxiety of the mice, an open field test was performed. (1) First, 30 minutes before performing the open field test, the chiral gamma-lactam derivative of the present disclosure or a pharmaceutically acceptable salt thereof and a vehicle (saline, test drug) were injected intraperitoneally to the mice and their behavior was video recorded for 10 minutes. (2) Video analysis was used to check for sedation and measure the distance the mice moved.

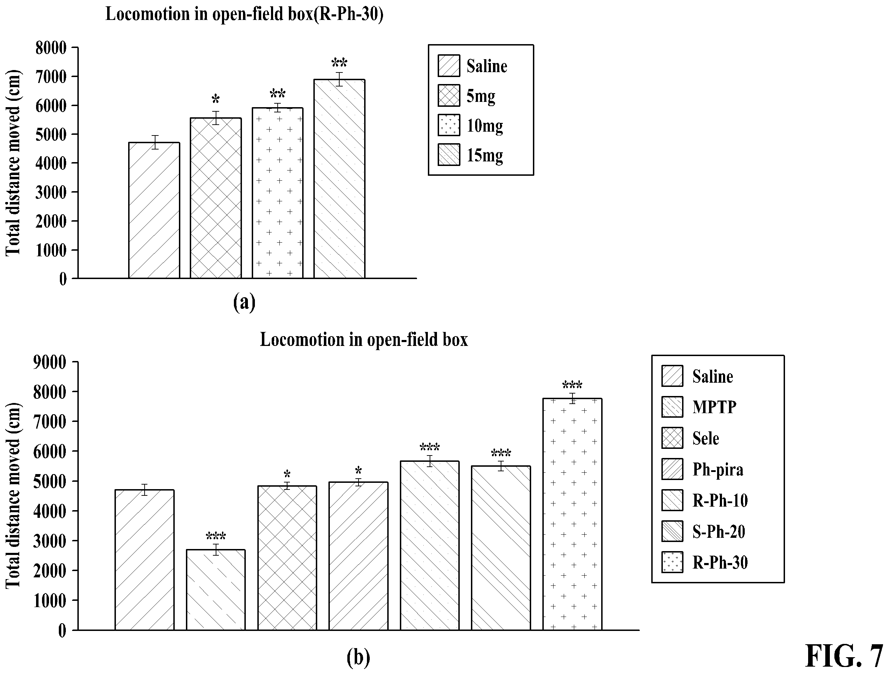

When the mice were treated with Ph-30 compound at concentrations of 5, 10, and 15 mg/kg, the distance they moved increased compared to the control group at all concentrations. There was a difference in the total distance moved depending on the concentration, but 10 mg/kg was used in subsequent experiments since too low a concentration may not be effective (FIG. 7 (a)).

The MPTP alone treatment group was used as a negative control group, and the Selegiline treatment group was used as a positive control group. When the mice were treated with the chiral gamma-lactam derivative of the present disclosure or a pharmaceutically acceptable salt thereof (Ph-10, 20, 30), the total distance moved increased compared to the (R)-phenylpiracetam (Ph-pira) treatment group as well as the MPTP alone and Selegiline treatment groups. In particular, the Ph-30 treatment group showed levels approximately three times higher than the MPTP alone treatment group (FIG. 7 (b)). This suggests that the derivative of the present disclosure or a pharmaceutically acceptable salt thereof increases motility and has an anti-anxiety disorder effect.

### Experimental Example 2.2. Forced Swim Test (FST)

The forced swim test (FST) was performed to induce depressive behavior in mice. (1) First, a 2L beaker was filled with water (25±1°C) until the water was about 10 cm deep, and the mouse was allowed to swim. The mouse was returned to the breeding cage after 6 minutes. (2) The restraint stress group was confined in a restraint tube for 90 minutes until 60 minutes before the forced swim test was to be performed, and then the mouse was allowed to swim as mentioned in (1). Thereafter, the mouse was returned to the breeding cage. (3) In this case, the chiral gamma-lactam derivative of the present disclosure or a pharmaceutically acceptable salt thereof (saline, test drug) was injected into the abdominal cavity 30 minutes before the forced swim test was performed. Immobility time was measured for 4 minutes out of the 6 minutes and these measurements were compared between groups.

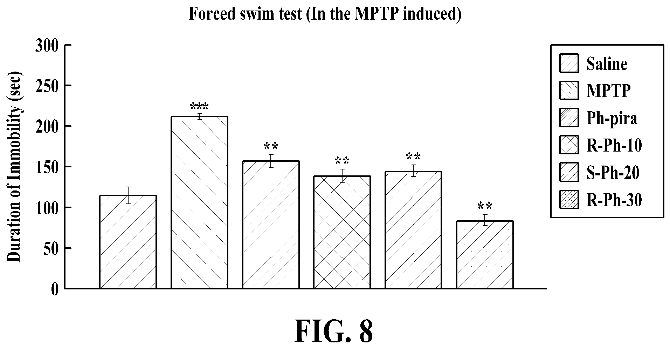

The results showed that the MPTP alone treatment group had an average of more than about twice the immobility time compared to the untreated control group (saline); the phenylpiracetam treatment group had a reduced immobility time compared to the MPTP alone treatment group, and , when treating was performed with the chiral gamma-lactam derivative of the present disclosure or a pharmaceutically acceptable salt thereof (Ph-10, 20, 30), the immobility time was much shorter. This is a statistically significant difference, indicating that the chiral gamma-lactam derivative of the present disclosure or a pharmaceutically acceptable salt thereof has an antidepressant effect. In particular, the Ph-30 compound group tended to show a lower immobility time than the untreated control group, suggesting that the Ph-30 compound has a particularly excellent antidepressant effect (FIG. 8).

### Experimental Example 2.3. Rotarod test

To evaluate the walking and motor abilities of the lower extremities of mice, a rotarod test was performed for 5 weeks. Motor coordination and balance were assessed by placing the mice on a rotating bar and measuring the time until they fell therefrom (latency to fall). MPTP was injected intraperitoneally into the mice at a concentration of 30 mg/kg for 5 consecutive days. One week after the MPTP was injected, MPTP was administered to the mice intraperitoneally (15 mg/kg) twice a week. The rotarod consists of a rotatable cylindrical rod with a diameter of 7 cm, located at a height of 60 cm and six baffles spaced at 15 cm intervals. Then, mice were placed on the rod while the rod was rotated for 180 seconds at a speed of 5 to 50 rpm (maximum speed) and the latency to fall was measured. At this time, the mice were sufficiently trained to be acclimated during the experimental period.

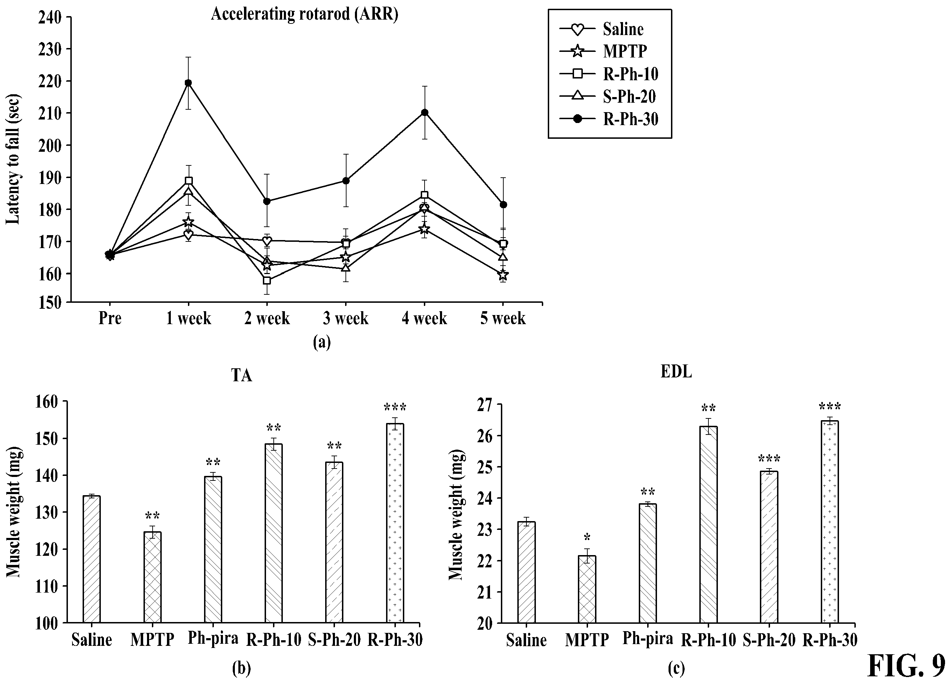

The results showed that, compared to the control group and the MPTP alone treatment group, motility was increased in all cases treated with the chiral gamma-lactam derivative of the present disclosure or a pharmaceutically acceptable salt thereof (Ph-10, 20, 30) (FIG. 9 (a)). In addition, after five weeks, both tibialis anterior (TA) and extensor digitorum longus (EDL) muscle weights were increased by statistically significant levels (FIGS. 9 (b) and 9 (c)). In particular, the Ph-30 compound showed approximately twice the efficacy compared to the Ph-20 compound, which is believed to be a result of the difference in chirality.

### Experimental Example 2.4. Evaluation of pilocarpine-induced seizures

A pilocarpine-induced seizure mouse model was created to study changes in brain activity. Pilocarpine is an agonist that binds to muscarinic cholinergic receptors in nerve cells and increases cell activity, and is used to create a mesial temporal lobe epilepsy model.

The present experiment was performed on surgically operated-on male C57BL/6J mice under a constant environmental condition (24-25°C, 50-60% humidity, 12-hour light/dark cycle). As a pre-treatment, methylscopolamine-bromide (1 mg/kg) was injected intraperitoneally into the mouse 30 minutes before pilocarpine was injected to suppress the side effects of pilocarpine, and the chiral gamma-lactam derivative of the present disclosure or a pharmaceutically acceptable salt thereof (15 mg) was injected intraperitoneally into the mouse 25 minutes before pilocarpine was injected. Pilocarpine (Sigma, Deisenhofen, Germany) was injected intraperitoneally into the mouse at a single dose of 330 mg/kg, and behavioral and electrophysiological seizure patterns were monitored for 24 hours. After several seizures, status epilepticus (SE) began.

The seizure patterns were divided into the following five types, and cases (4) and (5) were defined as convulsive seizures and analyzed for their frequency and duration: (0) no convulsive behavior; (1) facial clonus; (2) head nodding; (3) forelimb clonus; (4) rearing; animal in a standing posture aided by tail and the laterally spread hindlimbs showing increased tone; (5) rearing and falling back.

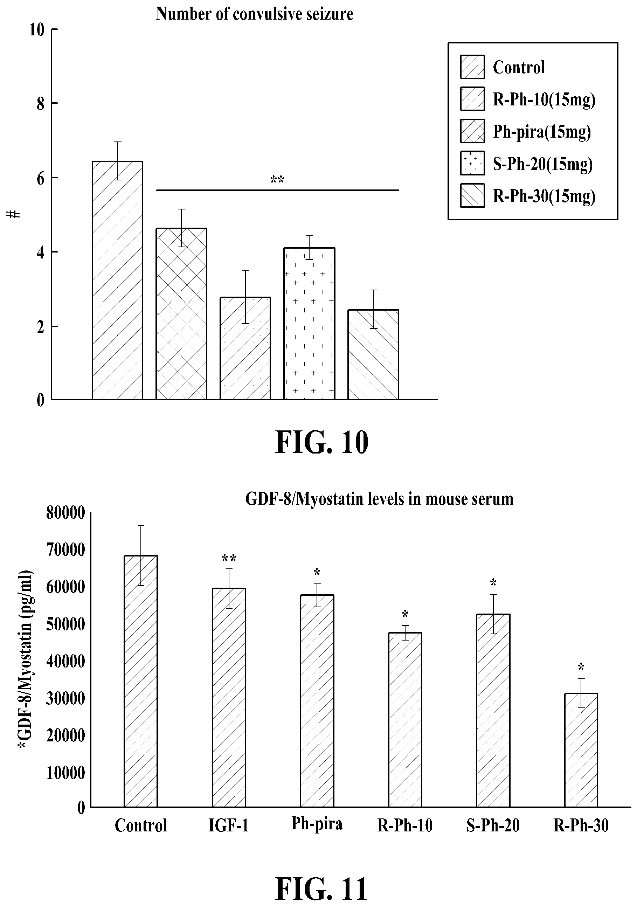

As shown in FIG. 10, the results showed that the total number of convulsive seizures was significantly lower for the Ph-30 compound than for the compounds of the control group, and the Ph-10 compound exhibited similar efficacy. In the case of the Ph-20 compound, the frequency was higher than for the Ph-30 and Ph-10 compound, but it was confirmed that the frequency was effectively reduced for the Ph-20 compound compared to the compounds of the control group or (R)-phenylpiracetam treatment group.

### Experimental Example 2.5. Confirming the changes in expression of markers related to sarcopenia

MPTP was injected intraperitoneally into the mouse at a concentration of 30 mg/kg for 5 consecutive days. One week after the MPTP was injected, the chiral gamma-lactam derivative of the present disclosure or a pharmaceutically acceptable salt thereof (Ph-10, 20, 30) was administered intraperitoneally to the mouse at a concentration of 15 mg/kg twice a week, for 5 weeks, and the changes in the expression level of GDF-8/Myostatine, a sarcopenia-related marker in mouse serum, were determined.

The expression levels of GDF-8/Myostatin in mouse serum were observed to be relatively lower in all of Ph-10, 20, and 30 compound groups at statistically significant levels compared to the IGF-1 and (R)-phenylpiracetam treatment groups. Similar to the cell experiments (Experimental Example 1.3.), it was confirmed through the mouse experiments that the compounds differ according to chirality, with the Ph-30 compounds having the lowest expression levels (FIG. 11). This suggests that the chiral gamma-lactam derivative of the present disclosure or a pharmaceutically acceptable salt thereof may be utilized as a therapeutic agent for treating muscular diseases such as sarcopenia.

Although a number of embodiments have been described with reference to limited drawings, one of ordinary skill in the art will recognize that various modifications and alterations may be made to these embodiments based on the above detailed description. For example, suitable results may be achieved if the described techniques are performed in a different order, and/or if components in a described system, architecture, device, or circuit are combined in a different manner, and/or replaced or supplemented by other components or their equivalents.

Therefore, other implementations, other examples, and equivalents to the claims are also within the scope of the following claims.

## Claims

1. A pharmaceutical composition for preventing or treating a muscular disease, a mental disease or a degenerative neurological disease, comprising as an active ingredient any one or more selected from a group consisting of a pharmaceutically acceptable salt of a chiral gamma-lactam derivative represented by the following [Formula 1a]; a pharmaceutically acceptable salt of a chiral gamma-lactam derivative represented by the following [Formula 1b]; a chiral gamma-lactam derivative represented by the following [Formula 1c] or a pharmaceutically acceptable salt thereof; and a combination thereof:

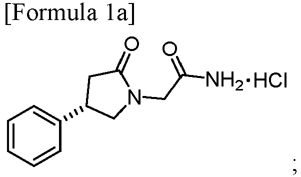

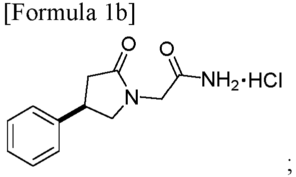

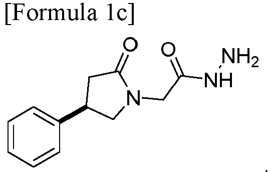

2. The pharmaceutical composition of claim 1, wherein the pharmaceutically acceptable salt of a chiral gamma-lactam derivative represented by the [Formula 1c] is a salt represented by the following [Formula 1d].

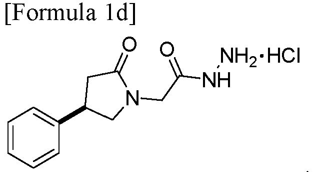

3. The pharmaceutical composition of claim 1, wherein the muscular disease is any one or more selected from the group consisting of atony, muscular atrophy, muscular dystrophy, myasthenia gravis, cachexia, rigid spinesyndrome, amyotrophic lateral sclerosis (Lou Gehrig's disease), Charcot-Marie-Tooth disease, sarcopenia, and a combination thereof.

4. The pharmaceutical composition of claim 1, wherein the mental disease is any one or more selected from the group consisting of depression, mania, bipolar disorder, schizophrenia, delirium, panic disorder, Attention-Deficit Hyperactivity Disorder (ADHD), cognitive disorder, anxiety disorder, Post-Traumatic Stress Disorder (PTSD), and a combination thereof.

5. The pharmaceutical composition of claim 1, wherein the degenerative neurological disease is any one or more selected from the group consisting of Alzheimer's disease, Parkinson's disease, Huntington's disease, Pick's disease, Creutzfeldt-Jakob disease, spinocerebellar degeneration, spinocerebellar ataxia, Friedreich's ataxia, Machado-Joseph disease, dystonia, progressive supranuclear palsy, senile dementia, dementia with Lewy bodies, frontotemporal dementia, vascular dementia, alcohol related dementia, pre-senile dementia, temporal lobe epilepsy, multiple sclerosis, cerebral amyloid angiopathy, systemic amyloidosis, Tourette's disorder, stroke, and a combination thereof.

6. A method for preparing a pharmaceutical composition for preventing or treating a muscular disease, a mental disease, or a degenerative neurological disease, which comprises a pharmaceutically acceptable salt of a chiral gamma-lactam derivative represented by [Formula 1a] as an active ingredient, comprising the following steps:
(1) Producing a compound represented by [Formula 4a] from a compound represented by [Formula 3a] using a catalyst compound represented by [Formula 2];
(2) Producing a compound represented by [Formula 5a] from the compound represented by [Formula 4a];
(3) Producing a compound represented by [Formula 6a] from the compound represented by [Formula 5a]; and
(4) Treating the compound represented by [Formula 6a] with sodium hydride (NaH), ethyl bromoacetate (BrCH₂COOEt), and ammonia (NH₃) to produce a compound represented by [Formula 1a]; 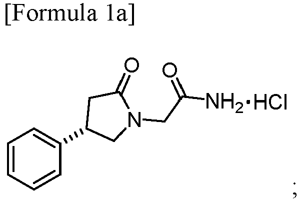 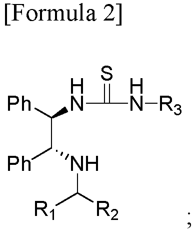 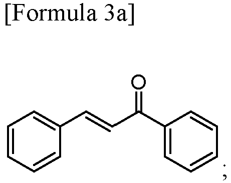 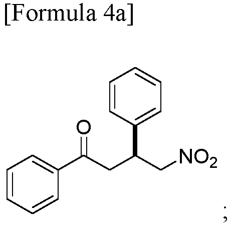 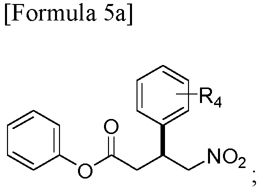 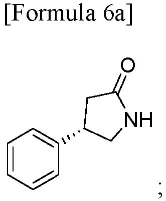
wherein, in the Formula 2, R₁ and R₂ are identical or different from each other, and are any one or more selected from the group consisting of hydrogen, a C₁-C₆ alkyl group, a halogen group, a cyano group, a nitro group, a trifluoromethyl group, an alkoxy group, a C₃-C₈ aryl group, and a combination thereof; R₃ is a C₃-C₈ aryl group, wherein the one or more hydrogen of the aryl group is unsubstituted or substituted with any one or more selected from the group consisting of a C₁-C₆ alkyl group, a halogen group, a cyano group, a nitro group, a trifluoromethyl group, an alkoxy group, and a combination thereof; and in the Formula 5a, R₄ is a hydrogen or a halogen group.

7. The method of claim 6, wherein the catalyst compound represented by the [Formula 2] is any one or more selected from the group consisting of catalyst compounds represented by the following [Formula 2-1] to [Formula 2-11] and a combination thereof. 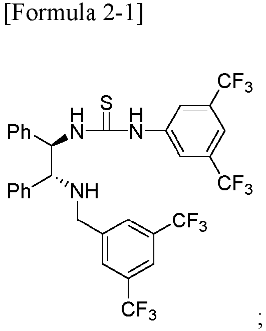 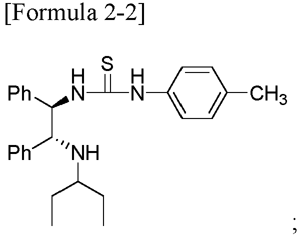 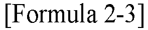 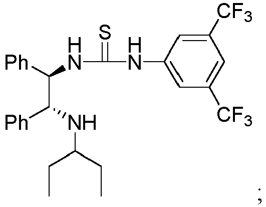 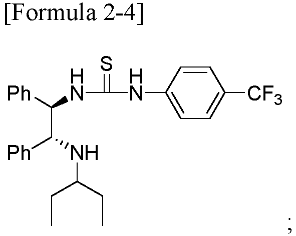 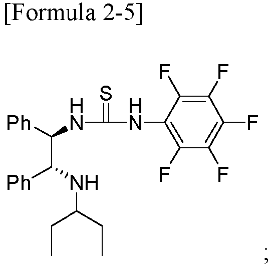 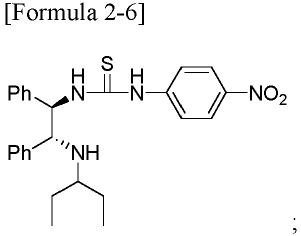 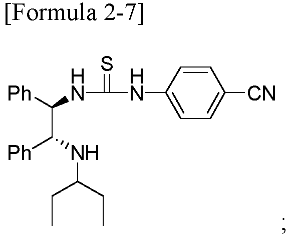 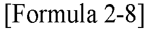 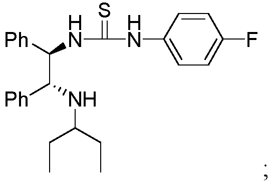 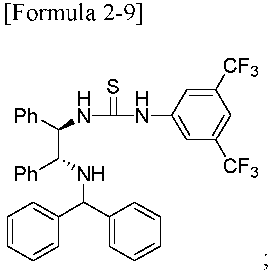 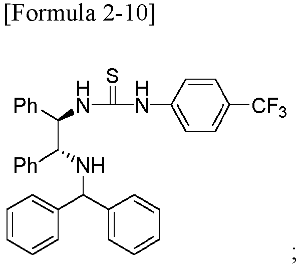 and

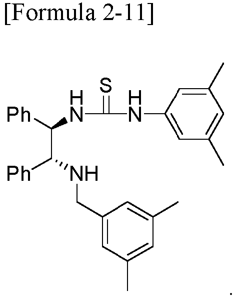

8. The method of claim 6, wherein the compound represented by the [Formula 5a] is any one or more selected from the group consisting of compounds represented by the following [Formula 5a-1] and [Formula 5a-2] and a combination thereof. 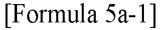 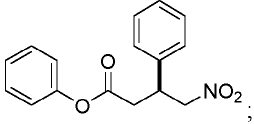 and

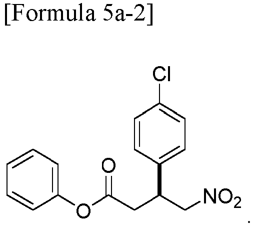

9. A method for preparing a pharmaceutical composition for preventing or treating a muscular disease, a mental disease, or a degenerative neurological disease, which comprises a pharmaceutically acceptable salt of a chiral gamma-lactam derivative represented by [Formula 1b] as an active ingredient, comprising the following steps:
(1) Producing a compound represented by [Formula 4b] from a compound represented by [Formula 3b] using a catalyst compound represented by [Formula 2];
(2) Producing a compound represented by [Formula 5b] from the compound represented by [Formula 4b];
(3) Producing a compound represented by [Formula 6b] from the compound represented by [Formula 5b]; and
(4) Treating the compound represented by [Formula 6b] with sodium hydride (NaH), ethyl bromoacetate (BrCH₂COOEt), and ammonia (NH₃) to produce a compound represented by [Formula 1b]; 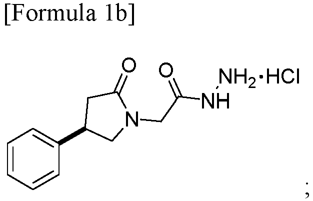 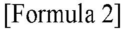 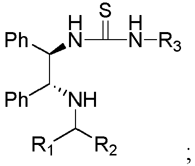 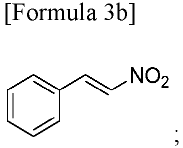 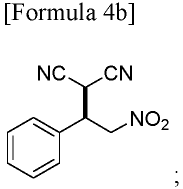 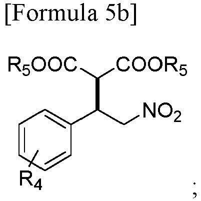 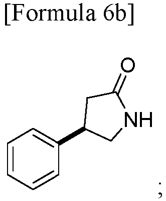
wherein, in the Formula 2, R₁ and R₂ are identical or different from each other, and are any one or more selected from the group consisting of hydrogen, a C₁-C₆ alkyl group, a halogen group, a cyano group, a nitro group, a trifluoromethyl group, an alkoxy group, a C₃-C₈ aryl group, and a combination thereof; R₃ is a C₃-C₈ aryl group, wherein the one or more hydrogen of the aryl group is unsubstituted or substituted with any one or more selected from the group consisting of a C₁-C₆ alkyl group, a halogen group, a cyano group, a nitro group, a trifluoromethyl group, an alkoxy group, and a combination thereof; and in the Formula 5b, R₄ is hydrogen or a halogen group, and R₅ is a C₁-C₆ alkyl group or benzyl group.

10. The method of claim 9, wherein the compound represented by the [Formula 5b] is any one or more selected from the group consisting of compounds represented by the following [Formula 5b-1] to [Formula 5b-8] and a combination thereof. 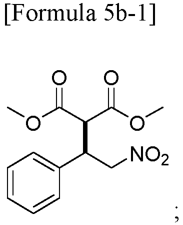 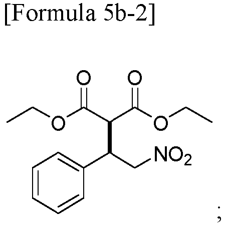 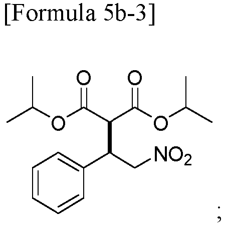 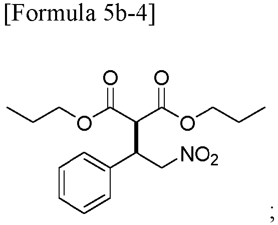 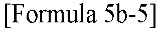 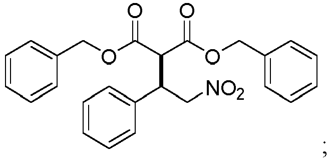 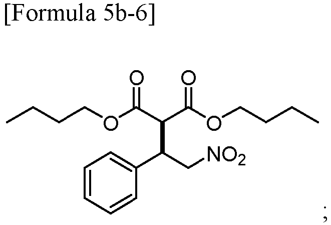 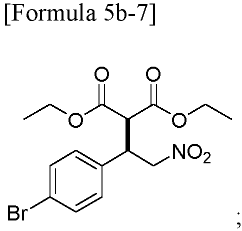 and

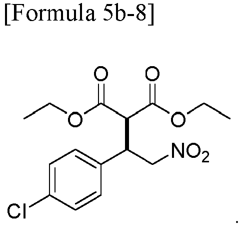

11. A method for preparing a pharmaceutical composition for preventing or treating a muscular disease, a mental disease, or a degenerative neurological disease, which comprises a pharmaceutically acceptable salt of a chiral gamma-lactam derivative represented by [Formula 1d] as an active ingredient, comprising the following steps:
(1) Producing a compound represented by [Formula 4b] from a compound represented by [Formula 3b] using a catalyst compound represented by [Formula 2];
(2) Producing a compound represented by [Formula 5b] from the compound represented by [Formula 4b];
(3) Producing a compound represented by [Formula 6b] from the compound represented by [Formula 5b]; and
(4) Treating the compound represented by [Formula 6b] with sodium hydride (NaH), ethyl bromoacetate (BrCH₂COOEt), and hydrazine hydrate to produce a compound represented by [Formula 1d]; 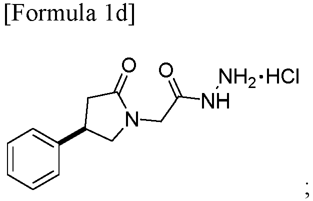 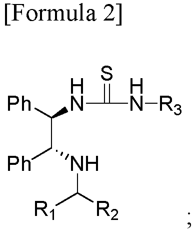 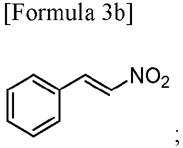 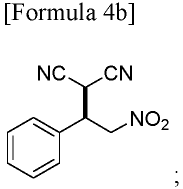 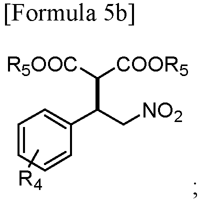 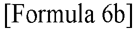 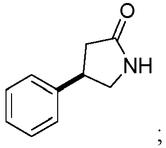
wherein, in the Formula 2, R₁ and R₂ are identical or different from each other, and are any one or more selected from the group consisting of hydrogen, a C₁-C₆ alkyl group, a halogen group, a cyano group, a nitro group, a trifluoromethyl group, an alkoxy group, a C₃-C₈ aryl group, and a combination thereof; R₃ is a C₃-C₈ aryl group, wherein the one or more hydrogen of the aryl group is unsubstituted or substituted with any one or more selected from the group consisting of a C₁-C₆ alkyl group, a halogen group, a cyano group, a nitro group, a trifluoromethyl group, an alkoxy group, and a combination thereof; and in the Formula 5b, R₄ is hydrogen or a halogen group, and R₅ is a C₁-C₆ alkyl group or benzyl group.
